# EUROPEAN PATENT APPLICATION

(11) **EP 4 831 610 A1**
(43) Date of publication of application: **28.10.2026**
(21) Application number: 24907662.1
(22) Date of filing: 06.08.2024
(51) Int. Cl.: G01N 15/06, G01N 15/0205, G01N 21/53, G01N 33/18, G01N 15/075, G01N 21/47

(54) **TURBIDITY MONITORING DEVICE**

(30) Priority: 20.12.2023 KR 20230186639
(71) Applicant: The Wave Talk, Inc., Daejeon 35240 (KR)
(72) Inventor: KIM, Young Dug, Seongnam-si, Gyeonggi-do 13597 (KR); CHO, Kyoung Man, Seoul 06224 (KR)
(74) Representative: AWA Sweden AB
(86) International application number: PCT/KR2024/011552
(87) International publication number: WO 2025/135376

(57) **Abstract**

A turbidity monitoring apparatus is provided. The turbidity monitoring apparatus includes a measurement container in which a fluid accommodation portion configured to accommodate a fluid to be measured is formed, the measurement container including an inlet pipe configured to supply the fluid to be measured to the fluid accommodation portion, and an outlet pipe configured to discharge the fluid to be measured to the outside, a wave source configured to irradiate a wave toward the fluid accommodation portion, and a detector configured to detect a laser speckle generated by multiple scattering of the irradiated wave in the fluid to be measured, wherein the measurement container includes a flow path formation portion formed on a side wall surface of the fluid accommodation portion, and configured to guide a flow of the fluid to be measured introduced through the inlet pipe, and the wave source and the detector are positioned adjacent to a surface of the measurement container on which the flow path formation portion is formed.

## Description

### Technical Field

The disclosure relates to a turbidity monitoring apparatus.

### Background Art

Turbidity is an index which quantitatively represents the degree of cloudiness of water and is a measure of resistance to light transmission. Turbidity is caused by various suspended materials, and the size of turbidity particles varies from colloidal dispersion to coarse dispersoids. Materials causing turbidity are extremely diverse, ranging from pure inorganic materials to primarily natural organic materials, and specifically, bacteria, microorganisms, and algae generated due to pure inorganic materials such as soil and sediments, natural organic materials, or large amounts of inorganic and organic materials introduced from industrial wastewater and domestic sewage also act as causative materials of turbidity.

A turbidity measurement apparatus is an essential element in water quality measurement systems for water supply and sewerage, and requires turbidity measurement over a wide range according to water quality specifications (raw water, settled water, treated water, pipe cleaning water, etc.). Turbidity measurement apparatuses for measuring drinking water quality may be classified into high-concentration turbidimeters for measuring high-concentration turbidity such as water supply source water and pipe cleaning discharge water, and low-concentration turbidimeters for measuring low-concentration turbidity such as treated tap water.

In the related art, as turbidity meters for measuring turbidity, mainly portable probe-type turbidity meters and integrated-structure turbidity meters installed on-site such as at water treatment plants have been used. To measure more accurate turbidity with the turbidimeters, the amount of fine bubbles included in a sample needs to be minimized, and the sample is not to be affected by external environmental conditions such as temperature and pressure.

In the related art, turbidity may be monitored by measuring the turbidity of continuously supplied fluid, for example, water, by using such turbidity measurement apparatuses. However, bubbles may be generated in a measurement space where the fluid stays for a certain time in the turbidity measurement apparatus, and the generated bubbles may be trapped at corners of the measurement space and may also form on a light source portion and a camera area. Furthermore, there is a problem that the generated bubbles are not easily removed even when water is circulated, which makes accurate turbidity measurement difficult.

### Disclosure

### Technical Problem

The disclosure is to solve the above problems and to provide a turbidity monitoring apparatus having a structure which facilitates removal of bubbles generated in a measurement space of a turbidity measurement apparatus.

### Technical Solution

The turbidity monitoring apparatus includes a measurement container in which a fluid accommodation portion configured to accommodate a fluid to be measured is formed, the measurement container including an inlet pipe configured to supply the fluid to be measured to the fluid accommodation portion, and an outlet pipe configured to discharge the fluid to be measured to the outside, a wave source configured to irradiate a wave toward the fluid accommodation portion, and a detector configured to detect a laser speckle generated by multiple scattering of the irradiated wave in the fluid to be measured, wherein the measurement container includes a flow path formation portion formed on a side wall surface of the fluid accommodation portion, and configured to guide a flow of the fluid to be measured introduced through the inlet pipe, and the wave source and the detector are positioned adjacent to a surface of the measurement container on which the flow path formation portion is formed.

### Advantageous Effects

The turbidity monitoring apparatus according to the embodiments of the disclosure may accurately measure turbidity by easily removing bubbles generated in a measurement space.

The scope of the disclosure is not limited by such an effect.

### Description of Drawings

FIG. 1 is a diagram for explaining a measurement principle of a turbidity monitoring apparatus according to an embodiment of the disclosure.
FIG. 2 is a side cross-sectional view of a turbidity monitoring apparatus according to the related art.
FIG. 3 is a perspective view of a turbidity monitoring apparatus according to a first embodiment of the disclosure.
FIG. 4 is a plan view of the turbidity monitoring apparatus of FIG. 3.
FIG. 5 is a side cross-sectional view of the turbidity monitoring apparatus taken along a line I-I' of FIG. 4.
FIG. 6 is an enlarged view of FIG. 5.
FIG. 7 is a cross-sectional view of the turbidity monitoring apparatus taken along a line A-A' of FIG. 3.
FIG. 8 is a cross-sectional view of the turbidity monitoring apparatus taken along a line B-B' of FIG. 3.
FIG. 9 is a perspective view of a measurement assembly of the turbidity monitoring apparatus of FIG. 3.
FIG. 10 is a side view of the measurement assembly of FIG. 9.
FIG. 11 is a perspective view of a turbidity monitoring apparatus according to a second embodiment of the disclosure.
FIG. 12 is an exploded perspective view of the turbidity monitoring apparatus of FIG. 11 in an exploded state.
FIGS. 13 and 14 are cross-sectional views of the turbidity monitoring apparatus taken along a line C-C' of FIG. 11.
FIG. 15 is a plan view of a housing of the turbidity monitoring apparatus of FIG. 11.
FIG. 16 is a cross-sectional view of the turbidity monitoring apparatus taken along a line D-D' of FIG. 11.
FIG. 17 is a perspective view of a turbidity monitoring apparatus according to a third embodiment of the disclosure.
FIG. 18 is a perspective view of the turbidity monitoring apparatus of FIG. 17 in an opened state.
FIG. 19 is a perspective view illustrating a state in which the turbidity monitoring apparatus of FIG. 17 is coupled to a conduit.
FIG. 20 is a cross-sectional view of the conduit taken along a line E-E' of FIG. 19.
FIG. 21 is a front view of the turbidity monitoring apparatus of FIG. 17 from a different angle.
FIG. 22 is a side view of the turbidity monitoring apparatus of FIG. 17 from a different angle.
FIG. 23 is an exploded perspective view of a first body portion and some components of the turbidity monitoring apparatus of FIG. 17 in an exploded state.
FIG. 24 is a plan view illustrating a state in which the first body portion and a measurement assembly of the turbidity monitoring apparatus of FIG. 17 are coupled to each other.
FIG. 25 is a cross-sectional view of the turbidity monitoring apparatus taken along a line II-II' of FIG. 17.
FIG. 26 is a cross-sectional view of the turbidity monitoring apparatus taken along a line III-III' of FIG. 17.
FIG. 27 is a perspective view of a turbidity monitoring apparatus according to a fourth embodiment of the disclosure.
FIG. 28 is an exploded perspective view of a second body portion and some components of the turbidity monitoring apparatus of FIG. 27 in an exploded state.
FIG. 29 is a perspective view illustrating a state in which the second body portion and a measurement assembly of the turbidity monitoring apparatus of FIG. 27 are coupled to each other.
FIG. 30 is a plan view illustrating a state in which the second body portion and the measurement assembly of the turbidity monitoring apparatus of FIG. 27 are coupled to each other.
FIG. 31 is a cross-sectional view of the turbidity monitoring apparatus taken along a line II-II' of FIG. 27.
FIG. 32 is a cross-sectional view of the turbidity monitoring apparatus taken along a line III-III' of FIG. 27.

### Best Mode

According to an embodiment of the disclosure, a turbidity monitoring apparatus includes a measurement container in which a fluid accommodation portion configured to accommodate a fluid to be measured is formed, the measurement container including an inlet pipe configured to supply the fluid to be measured to the fluid accommodation portion, and an outlet pipe configured to discharge the fluid to be measured to the outside, a wave source configured to irradiate a wave toward the fluid accommodation portion, and a detector configured to detect a laser speckle generated by multiple scattering of the irradiated wave in the fluid to be measured, wherein the measurement container includes a flow path formation portion formed on a side wall surface of the fluid accommodation portion, and configured to guide a flow of the fluid to be measured introduced through the inlet pipe, and the wave source and the detector are positioned adjacent to a surface of the measurement container on which the flow path formation portion is formed.

According to an embodiment of the disclosure, a central axis of the inlet pipe and a central axis of the outlet pipe may be parallel to each other.

According to an embodiment of the disclosure, the flow path formation portion may be positioned in parallel with the inlet pipe and the outlet pipe.

According to an embodiment of the disclosure, a central axis of the inlet pipe may be closer to the flow path formation portion than a central axis of the outlet pipe.

According to an embodiment of the disclosure, the wave source may be positioned closer to an inlet portion of the inlet pipe than the detector.

According to an embodiment of the disclosure, the flow path formation portion may include a planar portion, a first curved portion extending from the planar portion toward the outlet pipe to form a curved surface, and a second curved portion extending from the planar portion toward the inlet pipe to form a curved surface.

According to an embodiment of the disclosure, the detector and the wave source may be positioned on the planar portion.

According to an embodiment of the disclosure, the first curved portion may be formed to have a longer curved surface length than a curved surface length of the second curved portion.

According to an embodiment of the disclosure, the flow path formation portion may be configured to form a flow of the fluid to be measured in the fluid accommodation portion to reduce bubble generation in the fluid accommodation portion and to reduce bubbles remaining in the fluid accommodation portion.

According to an embodiment of the disclosure, a turbidity monitoring apparatus includes a measurement container in which a housing having a fluid accommodation portion formed therein to accommodate a fluid to be measured, an inlet pipe configured to supply the fluid to be measured, and an outlet pipe configured to discharge the fluid to be measured to the outside are integrally formed as a single body, and a measurement assembly including a wave source configured to irradiate a wave toward the fluid accommodation portion, and a detector configured to detect a laser speckle generated by multiple scattering of the irradiated wave in the fluid to be measured, wherein central axes of the inlet pipe and the outlet pipe coincide with a central axis of the housing, and the wave source and the detector are positioned together on a side of the measurement container.

According to an embodiment of the disclosure, the housing, the inlet pipe, and the outlet pipe may be formed as a single conduit.

According to an embodiment of the disclosure, the measurement container may include a measurement assembly accommodation portion positioned on a side surface of the housing to accommodate the measurement assembly.

According to an embodiment of the disclosure, the measurement container may include an opening penetrating the housing and the measurement assembly accommodation portion.

According to an embodiment of the disclosure, the opening may be configured to form a first space having a certain depth corresponding to a distance from an inner surface of the housing to a bottom surface of the measurement assembly.

According to an embodiment of the disclosure, a longitudinal direction of the opening may be identical to a longitudinal direction of the housing, and a width of the opening may be smaller than an inner diameter of the housing, and a length of the opening may be greater than the width of the opening.

According to an embodiment of the disclosure, the measurement assembly may include a case configured to accommodate the wave source and the detector, and the case may include a plate positioned adjacent to the fluid accommodation portion.

According to an embodiment of the disclosure, the plate may include a light-transmitting area.

According to an embodiment of the disclosure, a turbidity monitoring apparatus includes a first body portion positioned to surround at least a portion of a conduit through which a fluid to be measured flows, a second body portion positioned to surround at least a portion of the conduit, the second body portion being separable from or couplable to the first body portion, and a measurement assembly positioned on the first body portion or the second body portion, the measurement assembly including a wave source configured to irradiate a wave toward the fluid to be measured, and a detector configured to detect a laser speckle generated by multiple scattering of the irradiated wave in the fluid, wherein the wave source and the detector are positioned together on one of the first body portion and the second body portion.

According to an embodiment of the disclosure, the first body portion and the second body portion may be connected to be relatively rotatable about a single axis.

According to an embodiment of the disclosure, the first body portion and the second body portion may be connected to each other via a hinge portion including the single axis and may be detachably coupled to each other via a fastening portion positioned on a side opposite the hinge portion.

According to an embodiment of the disclosure, the first body portion or the second body portion may form an opening corresponding to a portion in which the measurement assembly is positioned.

According to an embodiment of the disclosure, the opening may have a distance in a longitudinal direction greater than a distance in a width direction.

According to an embodiment of the disclosure, the longitudinal direction of the opening may be parallel to a longitudinal direction of the conduit.

According to an embodiment of the disclosure, the width direction of the opening may be parallel to the longitudinal direction of the conduit.

According to an embodiment of the disclosure, the wave source and the detector may be sequentially positioned in a direction parallel to the longitudinal direction of the conduit.

According to an embodiment of the disclosure, the wave source and the detector may be sequentially positioned in a direction perpendicular to the longitudinal direction of the conduit.

Other aspects, features, and advantages of the disclosure will become more apparent from the following drawings, claims, and detailed description of the disclosure.

### Mode for Invention

Hereinafter, the following embodiments will now be described in detail with reference to the accompanying drawings. When described with reference to the drawings, identical or corresponding elements will be given the same reference numerals, and redundant description of these elements will be omitted.

In the description of the disclosure, when it is determined that detailed descriptions of related well-known functions or components may unnecessarily obscure the gist of the embodiments of the disclosure, the detailed descriptions thereof will be omitted.

The embodiments may be subjected to various modifications, and specific embodiments are illustrated in the drawings and will be described in detail in the detailed description. Effects and features of the embodiments, and methods for achieving them will be clarified with reference to contents described below in detail with reference to the drawings. However, the embodiments may have different forms and should not be construed as being limited to the descriptions set forth herein.

In the drawings, portions unrelated to the description have been omitted for clarity of explanation of the disclosure, and similar portions are denoted by similar reference numerals throughout the specification.

In the following embodiments, it will be understood that although the terms "first" and "second" may be used to describe various elements, these elements should not be limited by these terms and these terms are only used to distinguish an element from another.

In the following embodiments, the singular forms include the plural forms unless the context clearly indicates otherwise.

In the following embodiments, it will be understood that the terms "comprise," "include," and "have" used herein specify the presence of stated features or elements, but do not preclude the presence or addition of one or more other features or elements.

In the following embodiments, it will be further understood that, when a unit, area, or element is referred to as being "on" another unit, area, or element, it may be directly or indirectly on the other unit, area, or element. That is, for example, intervening units, areas, or elements may be present.

In the following embodiments, unless the terms "connect" and "couple" clearly mean otherwise in context, the terms not necessarily mean that two components are directly and/or fixedly connected to each other, and do not exclude the presence of another component between the two components.

Sizes of elements in the drawings may be exaggerated or reduced for convenience of description. For example, because sizes and thicknesses of elements in the drawings are arbitrarily illustrated for convenience of explanation, the following embodiments are not limited thereto.

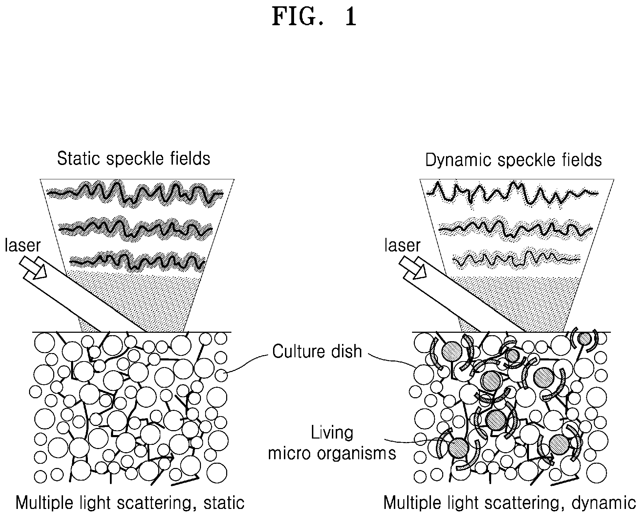

FIG. 1 is a diagram for explaining a measurement principle of a turbidity monitoring apparatus according to an embodiment of the disclosure.

Hereinafter, with reference to FIG. 1, the principle of monitoring turbidity of the disclosure will be described.

In the case of materials having homogeneous internal refractive index, such as glass, refraction may occur in a certain direction in case that light is irradiated. However, in case that coherent light such as laser is irradiated to a material having inhomogeneous internal refractive index, very complex multiple scattering may occur inside the material.

Referring to FIG. 1, among light or waves (hereinafter referred to as waves for simplification) irradiated from a wave source, a portion of the waves scattered through complex paths via multiple scattering may pass through a surface to be inspected. Waves passing through various points of the surface to be inspected may cause constructive interference or destructive interference with each other, and such constructive/destructive interference of waves may generate granular patterns (speckles).

In the specification, such waves scattered through complex paths may be referred to as "chaotic waves," and chaotic waves may be detected through laser speckles.

Again, the left drawing of FIG. 1 illustrates a case where a stable medium is irradiated with a laser. In case that a stable medium without movement of internal constituent materials is irradiated with coherent light (for example, laser), a stable speckle pattern without change may be observed.

However, as shown in the right drawing of FIG. 1, in case that an unstable medium having movement among internal constituent materials, such as bacteria, is included, the speckle pattern may change.

For example, an optical path may minutely change over time due to minute life activities of organisms (for example, intracellular movement, movement of microorganisms, movement of mites, or the like) or movement of fine turbidity materials in fluid. Because the speckle pattern is a phenomenon which occurs due to interference of waves, minute changes in the optical path may cause changes in the speckle pattern. Accordingly, by measuring temporal changes in the speckle pattern, movement of organisms or movement of fine turbidity materials in fluid may be rapidly measured. As described above, during measurement of changes in the speckle pattern over time, the presence of organisms and a concentration of turbidity materials may be determined, and furthermore, the type of organisms may also be determined.

In the specification, a component which measures such changes in the speckle pattern may be defined as a chaotic wave sensor.

In this case, the fluid may include liquid or gas. In addition, the fluid may include materials in which microorganisms may proliferate, and may include, for example, water such as tap water or sewage. The fluid may include suspended materials in water, which have a particle diameter of 2 µm or more and are not dissolved in water, or turbidity materials in water having a particle diameter of less than 2 µm.

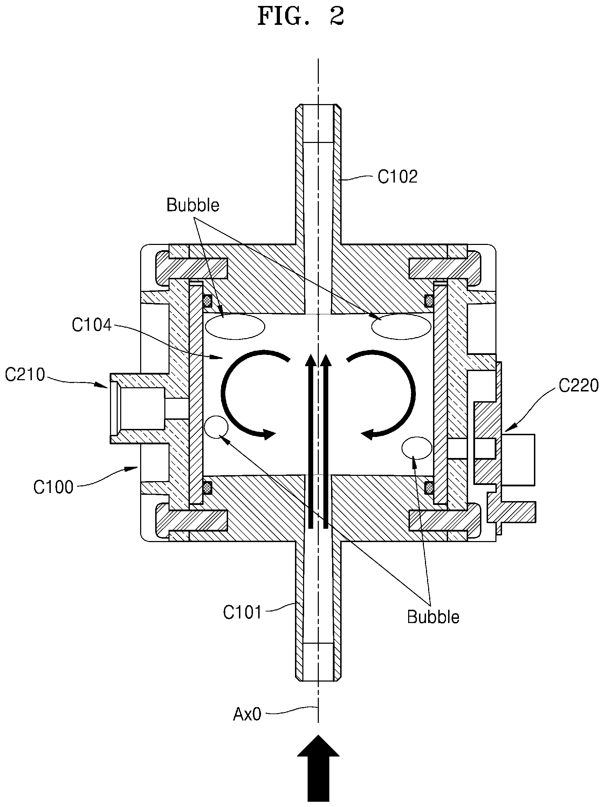

FIG. 2 is a side cross-sectional view of a turbidity monitoring apparatus according to a comparative embodiment. In the case of the turbidity monitoring apparatus according to the comparative example, an inlet pipe C101 and an outlet pipe C102 connected to a fluid accommodation portion C104 which accommodates a fluid to be measured may be formed on a same axis. In other words, a central axis of the inlet pipe C101 and a central axis of the outlet pipe C102 may be identical to each other. In addition, the inlet pipe C101 and the outlet pipe C102 may be formed to pass through a center of the fluid accommodation portion C104. To express this from another perspective, the fluid accommodation portion C104 which forms a space having a larger diameter than the inlet pipe C101 and the outlet pipe C102 may be connected between the inlet pipe C101 and the outlet pipe C102.

Also, with reference to a central axis Ax0 of the inlet pipe C101 and the outlet pipe C102, a wave source C210 may be positioned on a side wall surface of the fluid accommodation portion C104, and a detector C220 may be positioned on another side wall surface of the fluid accommodation portion C104. That is, the wave source C210 and the detector C220 may be positioned on different wall surfaces, and specifically, may be positioned on inner wall surfaces facing each other.

In addition, in the turbidity monitoring apparatus according to the comparative example, the fluid to be measured supplied from the inlet pipe C101 may enter the fluid accommodation portion C104, stay for a certain period of time, flow in an arrow direction shown in the drawing, and be discharged through the outlet pipe C102. Meanwhile, bubbles may be generated in the flowing fluid, and the fluid to be measured may enter the fluid accommodation portion C104 while including bubbles. In this case, the bubbles may remain in corner areas of the fluid accommodation portion C104 without disappearing. In addition, bubbles may adhere to portions in which the detector C220 and the wave source C210 are positioned among the inner wall surfaces of the fluid accommodation portion C104. Such bubbles may not be easily removed even when fluid enters and circulates, and may remain in the fluid accommodation portion C104, interfering with accurate turbidity measurement of the apparatus. The disclosure is to solve the above problems and to provide a turbidity monitoring apparatus in which the structure of a measurement container including an inlet pipe, an outlet pipe, and a fluid accommodation portion is improved, and the arrangement of a detector and a wave source is optimized to facilitate removal of internal bubbles and improve measurement accuracy.

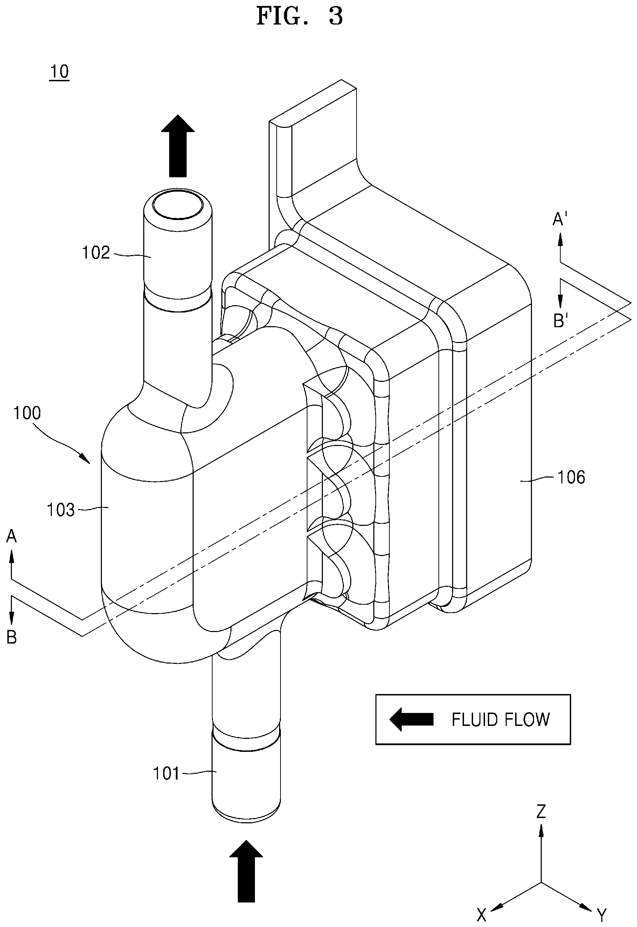

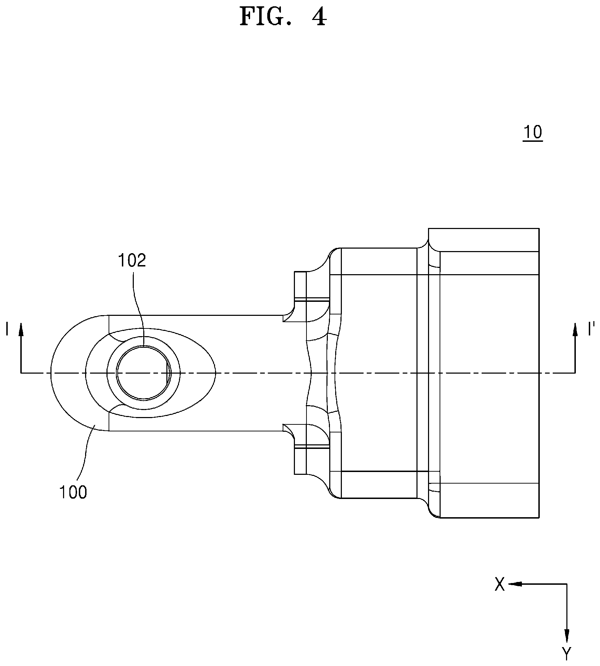

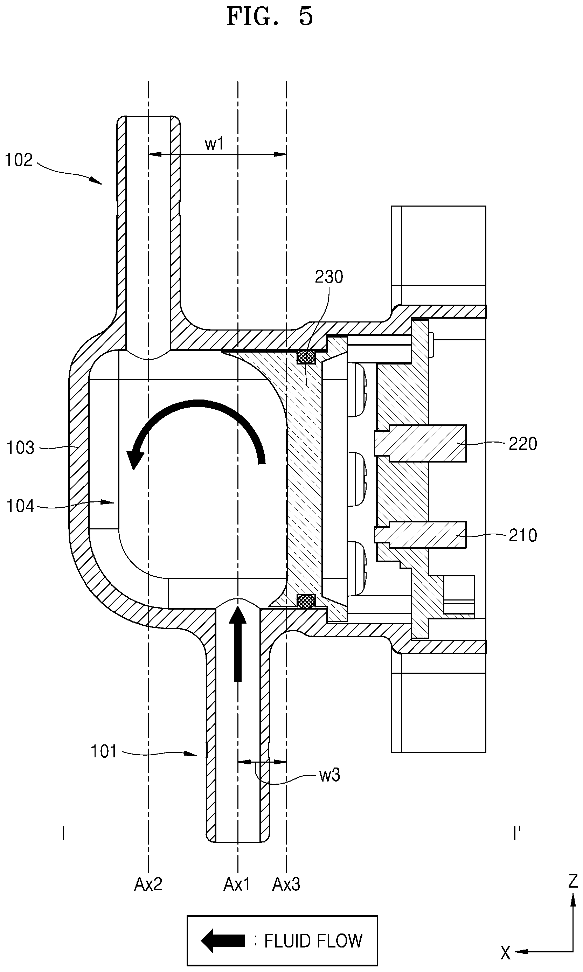

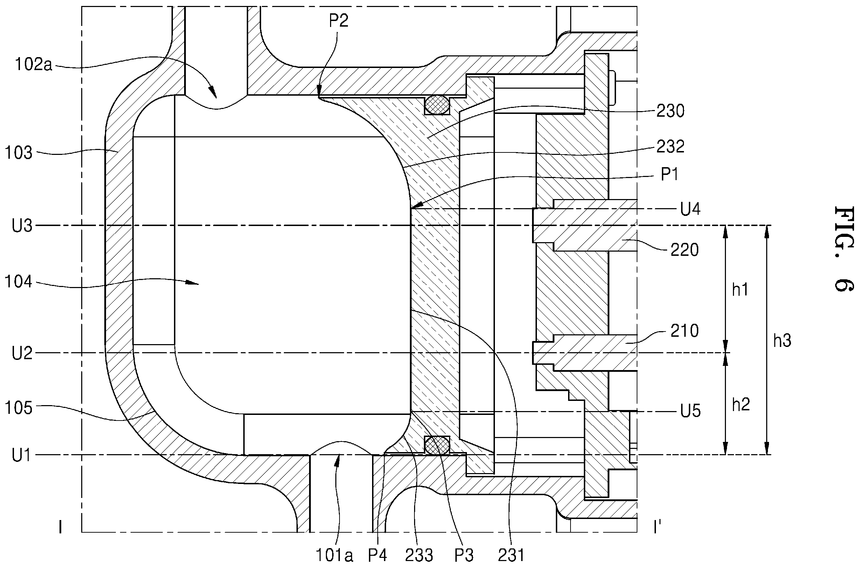

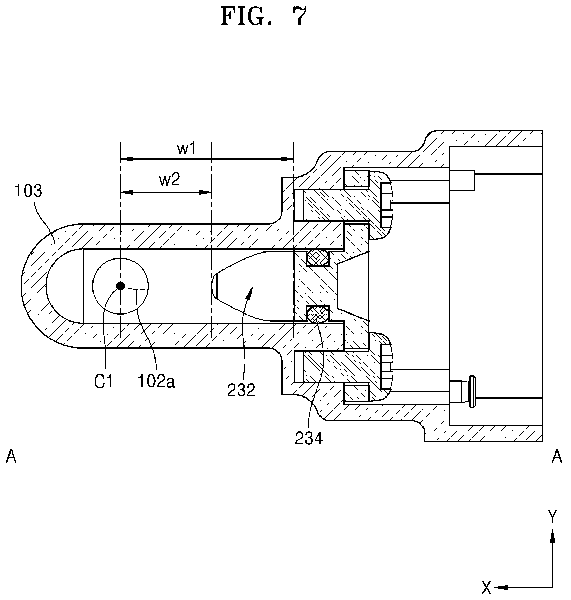

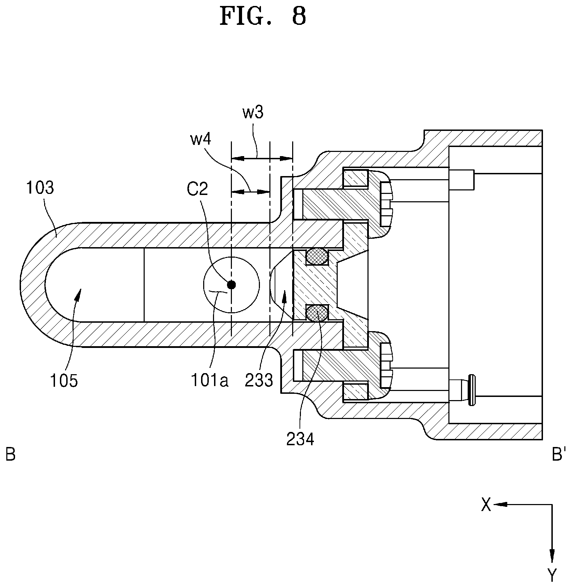

FIG. 3 is a perspective view of a turbidity monitoring apparatus 10 according to a first embodiment of the disclosure, and FIG. 4 is a plan view of the turbidity monitoring apparatus 10 of FIG. 3. FIG. 5 is a side cross-sectional view of the turbidity monitoring apparatus 10 taken along a line I-I' of FIG. 4, and FIG. 6 is an enlarged view of FIG. 5. FIG. 7 is a cross-sectional view of the turbidity monitoring apparatus 10 taken along a line A-A' of FIG. 3, and FIG. 8 is a cross-sectional view of the turbidity monitoring apparatus 10 taken along a line B-B' of FIG. 3.

First, referring to FIGS. 3 to 8, the turbidity monitoring apparatus 10 according to an embodiment of the disclosure may include a measurement container 100, a wave source 210, and a detector 220. In addition, although not shown in the drawings, the turbidity monitoring apparatus 10 may further include a controller (not shown). This will be described in detail below.

In this case, the measurement container 100 may be surrounded by a housing 103 and have formed therein a fluid accommodation portion 104 which accommodates a fluid to be measured, and may include an inlet pipe 101 which supplies fluid to the fluid accommodation portion 104 and an outlet pipe 102 which discharges fluid to the outside. In addition, the measurement container 100 may further include a flow path formation portion 230 which is formed on a side wall surface of the fluid accommodation portion 104.

The measurement container 100 may have formed therein the fluid accommodation portion 104 having a certain volume such that the inlet pipe 101 and the outlet pipe 102 may each be connected to the fluid accommodation portion 104. For example, the inlet pipe 101 and the outlet pipe 102 may be formed to communicate with the fluid accommodation portion 104. To express this from another perspective, the fluid accommodation portion 104 which forms a space having a larger diameter than the inlet pipe 101 and the outlet pipe 102 may be connected between the inlet pipe 101 and the outlet pipe 102.

The fluid accommodation portion 104 may be formed such that all surfaces except for the inlet pipe 101 and the outlet pipe 102 are closed, or may have a shape in which at least one surface is open. In this case, the flow path formation portion 230 to be described below may be positioned on the open surface to close the open surface of the fluid accommodation portion 104.

In addition, the inlet pipe 101 may be a tubular member having a certain inner diameter and may be connected to the outside to supply the fluid to be measured toward the fluid accommodation portion 104. Similarly, the outlet pipe 102 may be a tubular member having a certain inner diameter and may be connected to the outside to discharge fluid from the fluid accommodation portion 104 and transfer the fluid to the outside.

In this case, the inlet pipe 101, the outlet pipe 102, and the housing 103 may be integrally formed as a single body. In other words, an inner surface of the inlet pipe 101 may be connected to an inner surface of the fluid accommodation portion 104, and an inner surface of the fluid accommodation portion 104 may be connected to an inner surface of the outlet pipe 102. In this case, a portion in which the inlet pipe 101 is connected to the fluid accommodation portion 104 may be referred to as an inlet portion 101a, and a portion in which the outlet pipe 102 is connected to the fluid accommodation portion 104 may be referred to as an outlet portion 102a.

In addition, a central axis of the inlet pipe 101 and a central axis of the outlet pipe 102 may be parallel to each other. For example, a central axis Ax1 of the inlet pipe 101 and a central axis Ax2 of the outlet pipe 102 may not be positioned on a same axis. Specifically, the inlet pipe 101 may be formed on a side of the fluid accommodation portion 104, and the outlet pipe 102 may be formed on another side opposite the surface on which the inlet pipe 101 is formed. For example, the fluid accommodation portion 104 may be positioned between the inlet pipe 101 and the outlet pipe 102.

In this case, the flow path formation portion 230 may be positioned on a side wall surface of the fluid accommodation portion 104, and the flow path formation portion 230 may be positioned parallel to the inlet pipe 101 and the outlet pipe 102. In other words, a virtual axis Ax3 passing through a planar portion 231 formed by the flow path formation portion 230 may be positioned to be parallel to the central axis Ax1 of the inlet pipe 101 and the central axis Ax2 of the outlet pipe 102. However, the spirit of the disclosure is not necessarily limited thereto, and the planar portion 231 of the flow path formation portion 230 may form a certain angle with the central axis Ax1 of the inlet pipe 101 or the central axis Ax2 of the outlet pipe 102.

Also, the central axis Ax1 of the inlet pipe 101 may be closer to the flow path formation portion 230 than the central axis Ax2 of the outlet pipe 102. For example, as shown in FIGS. 5 and 7, a distance w3 from the planar portion 231 of the flow path formation portion 230 to the central axis Ax1 of the inlet pipe 101 may be shorter than a distance w1 from the planar portion 231 of the flow path formation portion 230 to the central axis Ax2 of the outlet pipe 102. Accordingly, fluid supplied from the inlet pipe 101 may flow toward a side adjacent to the flow path formation portion 230.

Moreover, the flow path formation portion 230 may include not only the planar portion 231 but also a first curved portion 232 and a second curved portion 233. In this case, the first curved portion 232 may be a portion which extends from the planar portion 231 toward the outlet pipe 102 to form a curved surface, and the second curved portion 233 may be a portion which extends from the planar portion 231 toward the inlet pipe 101 to form a curved surface.

In this case, the planar portion 231 may be a main area which forms a side wall surface of the fluid accommodation portion 104, and the first curved portion 232 and the second curved portion 233 may be portions which form corner portions of the fluid accommodation portion 104.

Specifically, referring to FIG. 6, a portion from a position P1 at which a curved surface starts in the planar portion 231 to a position P2 in contact with the inner surface of the fluid accommodation portion 104 may be referred to as the first curved portion 232. For example, an end P1 of the first curved portion 232 may be connected to the planar portion 231, and another end P2 may be connected to the inner surface of the fluid accommodation portion 104. Also, the other end P2 of the first curved portion 232 of the flow path formation portion 230 may be formed adjacent to the outlet portion 102a. To express this from another perspective, a distance w2 from the other end P2 of the first curved portion 232 to the central axis Ax2 of the outlet pipe 102 may be formed to be shorter than the distance w1 from the planar portion 231 of the flow path formation portion 230 to the central axis Ax2 of the outlet pipe 102.

In addition, a portion from a position P3 at which another curved surface starts in the planar portion 231 to a position P4 in contact with the inner surface of the fluid accommodation portion 104 may be referred to as the second curved portion 233. For example, an end P3 of the second curved portion 233 may be connected to the planar portion 231, and another end P4 thereof may be connected to the inner surface of the fluid accommodation portion 104. Also, the second curved portion 233 of the flow path formation portion 230 may be formed adjacent to the inlet portion 101a. To express this from another perspective, a distance w4 from the other end P4 of the second curved portion 233 to the central axis Ax1 of the inlet pipe 101 may be formed to be shorter than the distance w3 from the planar portion 231 of the flow path formation portion 230 to the central axis Ax1 of the inlet pipe 101.

Furthermore, the first curved portion 232 may be formed to have a longer curved surface length than the second curved portion 233. Because the inlet pipe 101 is formed closer to the flow path formation portion 230 than the outlet pipe 102, the second curved portion 233 may be formed adjacent to the inlet portion 101a and may have a relatively shorter curved surface length than the first curved portion 232. In other words, the first curved portion 232 may be formed adjacent to the outlet portion 102a and thus may have a relatively longer curved surface length than the second curved portion 233, and may be formed to pass through the central axis Ax1 of the inlet pipe 101. However, the spirit of the disclosure is not necessarily limited thereto, and lengths or shapes of the planar portion 231, the first curved portion 232, and the second curved portion 233 may be formed in various manners.

In other embodiments, in addition to the curved surface portions formed on the flow path formation portion 230, corners of the inner surface of the fluid accommodation portion 104 may form curved surface portions. For example, as shown in FIG. 6, a third curved portion 105 may be formed on the inner surface of the fluid accommodation portion 104 positioned diagonally with respect to the first curved portion 232.

As described above, the fluid accommodation portion 104 includes the flow path formation portion 230 on which curved surface portions are formed, and the inlet portion 101a is positioned adjacent to the flow path formation portion 230, and accordingly, the fluid to be measured may be introduced toward the flow path formation portion 230, and the flow path formation portion 230 may guide fluid flow such that the introduced fluid flows along the curved surface portions. For example, the fluid flowing into the fluid accommodation portion 104 may form a fluid flow along the curved surface portions of the flow path formation portion 230.

In addition, the measurement container 100 according to an embodiment of the disclosure may remove bubbles staying at corners of the fluid accommodation portion 104 by forming a fluid flow in the fluid accommodation portion 104 and discharge the bubbles through the outlet pipe 102, and may facilitate removal of bubbles adhering to the flow path formation portion 230. For example, the turbidity monitoring apparatus 10 according to an embodiment of the disclosure may reduce bubble generation in the fluid accommodation portion 104 and reduce bubbles remaining in the fluid accommodation portion 104.

Meanwhile, the measurement container 100 may include a mounting portion 106 for fixing the turbidity monitoring apparatus 10 according to an embodiment of the disclosure to an external apparatus. In this case, the mounting portion 10 may be integrally formed as a single body with the measurement container 100. The mounting portion 106 may form a space which accommodates PCB components including the wave source 210 and the detector 220 therein. In addition, the mounting portion 106 may form a path for inserting the measurement assembly 200 into the measurement container 100 and simultaneously form an accommodation portion which accommodates the measurement assembly 200.

Hereinafter, the wave source 210 and the detector 220 for turbidity measurement in the turbidity monitoring apparatus 10 according to an embodiment of the disclosure will be described.

The wave source 210 may irradiate a wave having coherence toward the fluid accommodation portion 104. In this case, the wave source 210 may apply all types of source apparatuses which may generate a wave, and may include a laser which may irradiate light in a specific wavelength band.

In this case, the wave source 210 may use a laser having good coherence to form a speckle which is an interference pattern in fluid flowing through an inner pipe. In this case, the shorter a spectral bandwidth of a light source which determines the coherence of a laser light source, the more the measurement accuracy may increase.

For example, the longer the coherence length, the more the measurement accuracy may increase. Accordingly, laser light having a spectral bandwidth of the wave source 210 less than a predefined reference bandwidth may be used as the wave source 210, and the shorter the spectral bandwidth is than the reference bandwidth, the more the measurement accuracy may increase. For example, the spectral bandwidth of the light source may be set such that the condition of Equation 1 below is maintained. [Equation 1] $Spectral bandwidth < 5 nm$

According to Equation 1, to measure a change in a laser speckle pattern, a spectral bandwidth of the wave source 210 may be maintained at less than 5 nm in case that light is irradiated to the fluid accommodation portion 104.

Furthermoe, the detector 220 may detect, at each of a plurality of preset time points, laser speckles generated by the irradiated wave undergoing multiple scattering in the fluid accommodation portion 104. The detector 220 may be positioned on the fluid accommodation portion 104. Specifically, the detector 220 may be positioned adjacent to a flow path formation portion 230. The detector 220 may include a charge-coupled device (CCD) camera. The detector 220 may measure an optical image emitted from the fluid accommodation portion 104 and provide the optical image to a controller (not shown).

In this case, the term "time point" refers to a specific moment in the flow of continuous time, and time points may be predetermined at equal time intervals. However, the time points are not necessarily limited thereto, and may be predetermined at arbitrary time intervals.

For example, in case that a light source in a visible wavelength band is used, the CCD camera, which is an imaging apparatus for capturing images, may be used. The detector 220 may detect laser speckles at at least a first time point, detect laser speckles at a second time point, and provide the laser speckles to the controller. In other embodiments, the first time point and the second time point are merely examples for the convenience of description, and the detector 220 may detect laser speckles at a plurality of time points greater in number than the first time point and the second time point.

In case that a wave is irradiated to the fluid in the fluid accommodation portion 104, the incident wave may form laser speckles through multiple scattering in the fluid. Because the laser speckles are generated by an interference phenomenon of light, in case that turbidity materials are constant in the fluid, a consistent interference pattern may appear over time.

In comparison, in case that a change in the turbidity materials occurs in the fluid, the laser speckles may change over time due to the change in the turbidity materials. The detector 220 may detect such time-varying laser speckles at each of the preset time points and provide the laser speckles to the controller.

The detector 220 may be required to allow high-speed measurement to measure turbidity from flowing fluid. In this case, the term "high-speed measurement" refers to detecting laser speckles faster than a flow speed of fluid. For example, a measurement speed of the detector 220 may be set to be faster than a flow speed of the fluid flowing in the fluid accommodation portion 104.

Furthermore, in case that an image sensor is used in the detector 220, the image sensor may be positioned such that a size d of a pixel of the image sensor becomes smaller than or equal to a grain size of the speckle pattern. For example, an image sensor in an optical system included in the detector 220 may be positioned to satisfy the condition of Equation 2 below. [Equation 2] $d \leq speckle grain size$

As shown in Equation 2, the size d of a pixel of the image sensor may be smaller than or equal to the grain size of the speckle pattern. However, in case that the pixel size becomes too small, undersampling may occur, which may cause difficulty in utilizing pixel resolution. Accordingly, to achieve an effective signal-to-noise ratio (SNR), the image sensor may be positioned such that no more than five pixels are positioned within a speckle grain size.

The controller may estimate, in real time, a concentration of suspended materials or turbidity materials in the fluid to be measured by using the detected laser speckles. The controller may estimate, in real time, the concentration of suspended materials or turbidity materials in the fluid based on an obtained temporal correlation. In the specification, the term "real time" refers to estimating the concentration within 3 seconds, and desirably, within 1 second.

In an embodiment, the controller may estimate the concentration of suspended materials or turbidity materials in the fluid by using a difference between first image information of laser speckles detected at the first time point and second image information of laser speckles detected at the second time point, which is different from the first time point.

In this case, the first image information and the second image information may include at least one of pattern information of the laser speckles and intensity information of the wave. In other embodiments, an embodiment of the disclosure is not limited to using only the difference between the first image information at the first time point and the second image information at the second time point, and may be extended to use image information of a plurality of laser speckles at a plurality of time points.

The controller may calculate a temporal correlation coefficient between images by using image information of laser speckles generated at each of the plurality of preset time points, and may estimate the concentration of suspended materials or turbidity materials in the fluid based on the temporal correlation coefficient. A temporal correlation of the detected laser speckle images may be calculated by using Equation 3 below. However, Equation 3 is merely an example, and it is obvious that the temporal correlation may be derived by using other equations. [Equation 3] $\overline{C} \left(x, y, τ\right) = \frac{1}{T - τ} {\sum }_{t = 1}^{T - τ} \overline{I} \left(x, y, t\right) \overline{I} \left(x,y;t+τ\right) δt$

In Equation 3, *c̅* represents a temporal correlation coefficient, *I̅* denotes a normalized light intensity, (x, y) indicates pixel coordinates of a camera, t denotes a measured time, T denotes a total measurement time, and τ represents a time lag.

According to Equation 3, the temporal correlation coefficient may be calculated, and in an embodiment, the concentration of suspended materials or turbidity materials in the fluid may be estimated through analysis in which the temporal correlation coefficient drops below a preset reference value. In addition, the controller may estimate the concentration of suspended materials or turbidity materials in the fluid by using a rate of change or a peak value of the temporal correlation coefficient.

In another embodiment, the controller may obtain a spatial correlation of an interference pattern. In this case, the spatial correlation given by the following equation may numerically indicate, within a certain range, how similar in brightness an arbitrary pixel and a pixel positioned at a distance r from the arbitrary pixel are on an image measured at time t. The certain range may be from -1 to 1. For example, the spatial correlation represents a degree of correlation between an arbitrary pixel and another pixel, where a value of 1 indicates a positive correlation, -1 indicates a negative correlation, and 0 indicates no correlation. Specifically, before the interference pattern is formed, the brightness is uniformly emitted, and thus, a spatial correlation of a sample image may show a positive correlation close to 1. However, once the interference pattern is formed, the correlation value may decrease toward 0.

In the detector 220, a brightness measured at time t at a pixel at a position r' = (x, y) may be defined as I(r', t), and a brightness of a pixel positioned at a distance r away may be defined as I(r' + r, t). By using this, the spatial correlation may be defined as shown in Equation 4 below. [Equation 4] $C \left(r, t\right) = \frac{1}{{C}_{0} \left(t\right)} ∬ I \left(r′+r,t\right) I \left(r′,t\right) dr ′$

*C*₀(*t*) was used to adjust a range of Equation 4 to be from -1 to 1. In case that a brightness I(r', t) measured at an arbitrary pixel at time t and a brightness I(r' + r, t) of a pixel positioned at a distance r are identical, a spatial correlation may result in 1, and in case that the brightnesses are not identical, the spatial correlation may have a value smaller than 1.

In an embodiment, the disclosure may represent the spatial correlation only as a function of time. To this end, the controller may calculate an average of the spatial correlation for pixels having a same distance r from the arbitrary pixel, as shown in Equation 5 below. [Equation 5] $C \left(ρ, t\right) = \frac{1}{2 π} {\int }_{0}^{2 π} C \left(r, t\right) dθ$

In an embodiment, the controller may substitute a preset distance into Equation 5 to represent the spatial correlation as a function of time, and by using this function, the degree to which the interference pattern is formed may be identified as a value within a certain range from 0 to 1.

The controller may determine concentration information of the suspended materials or the turbidity materials by using the spatial correlation as follows. The spatial correlation may be obtained by generating two overlapping identical images from a single image, shifting one of the two images in a direction by a preset distance, and analyzing how similar two adjacent pixels are between the shifted image and the unshifted image. In this case, the spatial correlation serves as an index indicating how uniform the images are. In case that an interference pattern is formed due to the suspended materials or the turbidity materials, the similarity between adjacent pixels may decrease due to a fine interference pattern, causing the spatial correlation value to also decrease.

Such a spatial correlation coefficient varies depending on the shift distance r. Within a certain distance range, the value decreases as the shift distance r increases, and when exceeding the certain distance range, the value becomes substantially constant. Therefore, to obtain a more meaningful spatial correlation, the controller may obtain the spatial correlation by shifting an image by a preset certain distance or more. In this case, the preset certain distance r depends on a speckle size, and when represented in pixel units, the controller may obtain the spatial correlation by shifting the image by a number of pixels greater than the speckle size.

In other embodiments, the controller may obtain not only the spatial correlation described above but also a temporal correlation of a measured interference pattern of the sample image, and detect the concentration of the suspended materials or the turbidity materials based on the obtained temporal correlation. The controller may calculate a temporal correlation coefficient between images by using image information of interference patterns measured in time series, and estimate the concentration of the suspended materials or the turbidity materials in the fluid based on the temporal correlation coefficient.

Hereinafter, an arrangement structure of the wave source 210 and the detector 220 in the measurement container 100 will be described in detail.

The wave source 210 and the detector 220 may be positioned in the measurement container 100 to face a side of the fluid accommodation portion 104. Specifically, the wave source 210 and the detector 220 may be positioned adjacent to a surface on which the flow path formation portion 230 is formed in the measurement container 100. For example, the wave source 210 may be positioned to irradiate a wave toward the fluid accommodation portion 104 passing through the flow path formation portion 230.

Specifically, an incidence hole (not shown) penetrating the fluid accommodation portion 104 may be formed in the flow path formation portion 230 to transmit a wave irradiated from the wave source 210 to the fluid, and the wave source 210 may be positioned to face the incidence hole. For example, the wave source 210 may be coupled to the incidence hole and positioned to irradiate a wave toward the fluid accommodation portion 104.

Meanwhile, the detector 220 may be positioned to detect laser speckles which are generated by multiple scattering in the fluid accommodation portion 104. Specifically, the flow path formation portion 230 may include an emission hole (not shown) penetrating the fluid accommodation portion 104 to guide a wave, which is emitted after being multiple-scattered in the fluid, toward the detector 220, and the detector 220 may be positioned to face the emission hole. For example, the detector 220 may be coupled to the emission hole and positioned to detect the wave.

The wave source 210 and the detector 220 may be positioned adjacent to each other. Specifically, the wave source 210 and the detector 220 may be positioned on a same printed circuit board (PCB).

In addition, the wave source 210 may be positioned on the planar portion 231 of the flow path formation portion 230. In other words, the wave source 210 may be positioned between the end P1 of the first curved portion 232 and the end P3 of the second curved portion 233.

Furthermore, both the wave source 210 and the detector 220 may be positioned on the planar portion 231 of the flow path formation portion 230. In other words, the wave source 210 and the detector 220 may be positioned between the end P1 of the first curved portion 232 and the end P3 of the second curved portion 233.

Also, the wave source 210 may be positioned closer to the inlet portion 101a of the inlet pipe 101 than the detector 220. In other words, a distance h2 between a virtual plane U2, in which the wave source 210 is positioned, and a virtual plane U1 including the inlet portion 101a may be shorter than a distance h3 between a virtual plane U3, in which the detector 220 is positioned, and the virtual plane U1 including the inlet portion 101a.

Meanwhile, a curved structure formed in the flow path formation portion 230 may potentially interfere with a light source emitted linearly from the wave source 210. Accordingly, as described above, positioning the wave source 210 closer to the inlet portion 101a than the detector 220 may help avoid interference by the first curved portion 232 during propagation of light from the wave source 210. The wave source 210 may also be positioned not to overlap with the second curved portion 233.

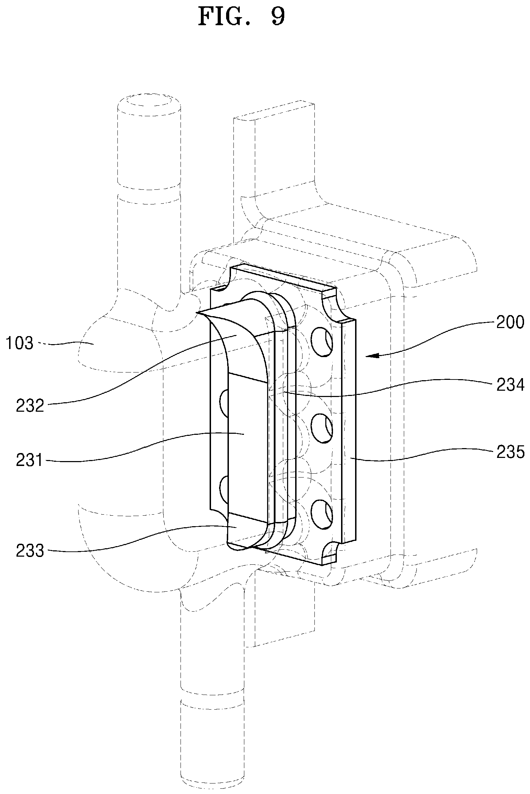

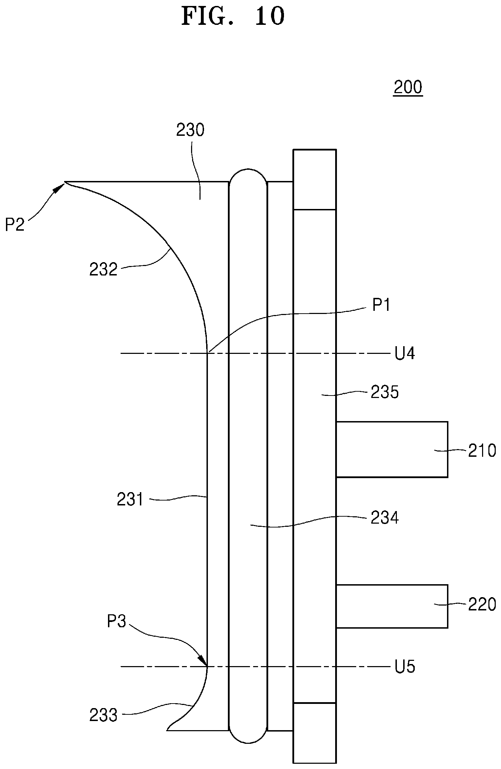

FIG. 9 is a perspective view of a measurement assembly 200 of the turbidity monitoring apparatus 10 of FIG. 3, and FIG. 10 is a side view of the measurement assembly 200 of FIG. 9.

Referring to FIGS. 9 and 10, the turbidity monitoring apparatus 10 according to an embodiment of the disclosure may include the measurement assembly 200. The measurement assembly 200 may include the flow path formation portion 230, a base 235, a sealing member 234, the wave source 210, and the detector 220. For example, the wave source 210 and the detector 220 may be coupled with the flow path formation portion 230 and provided as a semi-finished product.

Specifically, the base 235 may be a portion which is coupled to the measurement container 100 and may be integrally formed as a single body with the flow path formation portion 230. The base 235 may have the flow path formation portion 230 positioned on a side thereof, and the wave source 210 and the detector 220 coupled on another side thereof.

The sealing member 234 may be positioned along a periphery of the flow path formation portion 230. Specifically, the sealing member 234 may be positioned between the planar portion 231 of the flow path formation portion 230 and the base 235. In detail, the flow path formation portion 230 may have a groove formed along the periphery at a position adjacent to the base 235, and the sealing member 234 may be positioned in the groove. The sealing member 234 may serve to seal a side of the fluid accommodation portion 104 in case that the measurement assembly 200 is coupled to the measurement container 100.

By configuring the measurement assembly 200 in such a manner that the wave source 210 and the detector 220 are coupled with the base 235, an assembly process of the wave source 210 and the detector 220 may be facilitated. For example, by configuring the measurement assembly 200 separately, a process of assembling the wave source 210 and the detector 220 with the measurement container 100 may be simplified. In addition, the flow path formation portion 230 may be easily processed, and the wave source 210 and the detector 220 may be precisely positioned in the flow path formation portion 230.

In the following, the turbidity monitoring apparatus 20 according to a second embodiment of the disclosure will be described. In this case, the turbidity monitoring apparatus according to the second embodiment of the disclosure differs from the turbidity monitoring apparatus 10 according to the first embodiment of the disclosure described above with respect to the configuration of a measurement container 1100.

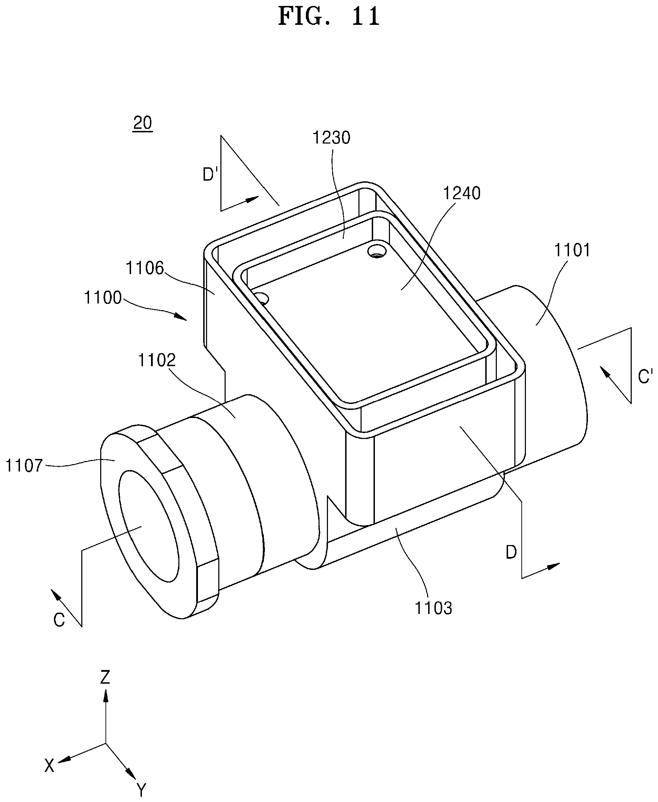

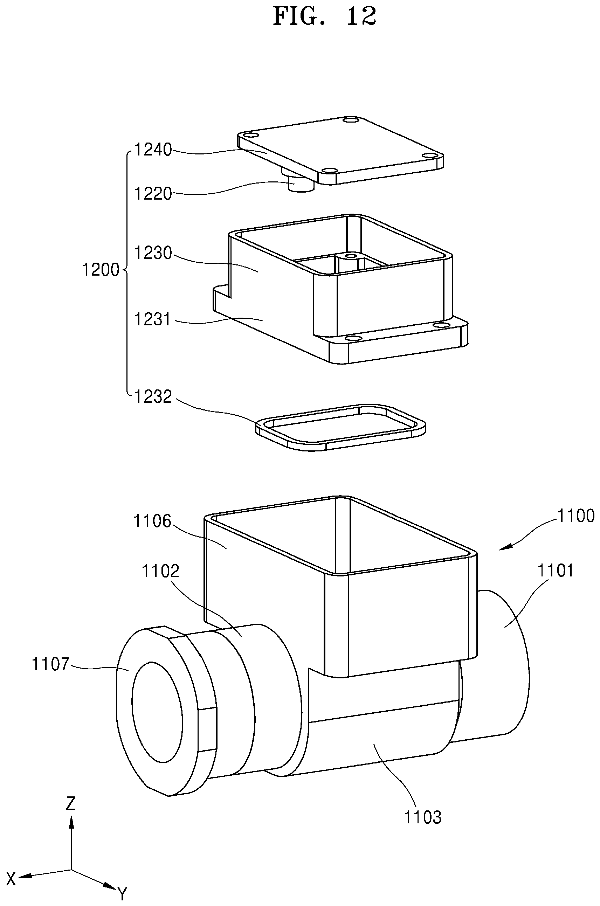

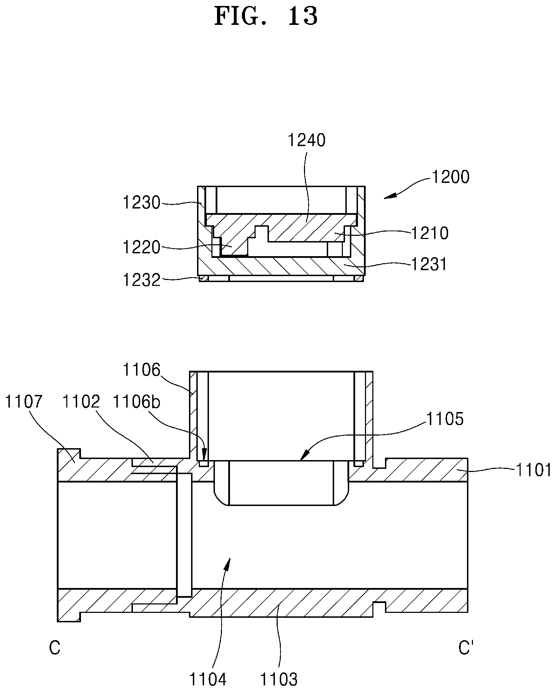

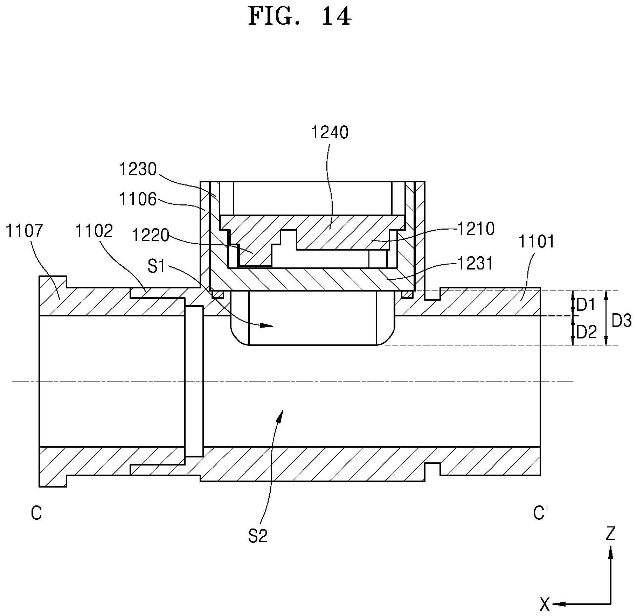

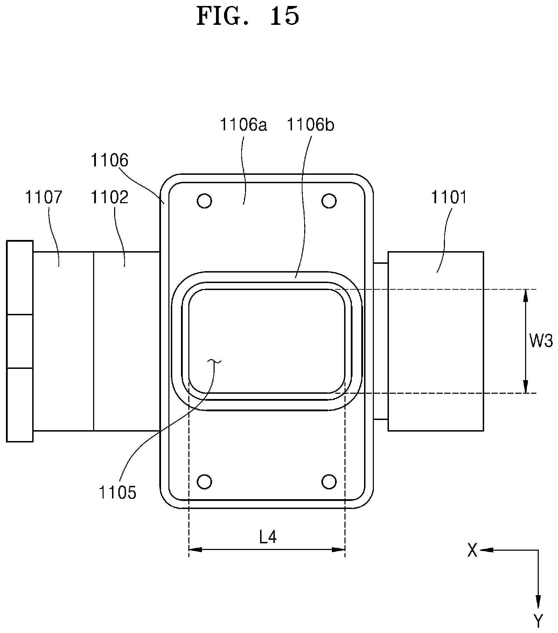

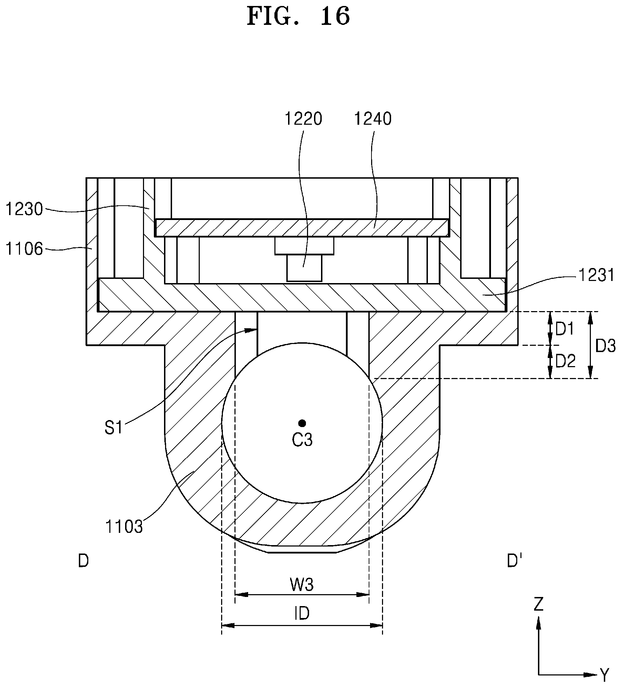

FIG. 11 is a perspective view of a turbidity monitoring apparatus 20 according to a second embodiment of the disclosure. FIG. 12 is an exploded perspective view of the turbidity monitoring apparatus 20 of FIG. 11 in an exploded state, and FIGS. 13 and 14 are cross-sectional views of the turbidity monitoring apparatus taken along a line C-C' of FIG. 11. FIG. 15 is a plan view of a housing 1103 of the turbidity monitoring apparatus 20 of FIG. 11, and FIG. 16 is a cross-sectional view of the turbidity monitoring apparatus taken along a line D-D' of FIG. 11.

Referring to FIGS. 11 to 14, the turbidity monitoring apparatus 20 according to the second embodiment of the disclosure may include the measurement container 1100 and a measurement assembly 1200. In this case, the measurement assembly 1200 may include a wave source 1210 and a detector 1220. In addition, although not shown in the drawings, the turbidity monitoring apparatus 20 may further include a controller (not shown).

Because the wave source 1210 and the detector 1220 according to the second embodiment of the disclosure are substantially identical to the wave source 210 and the detector 220 described in the first embodiment, a detailed description thereof will be omitted herein.

Hereinafter, the measurement container 1100 according to the second embodiment of the disclosure will be described in more detail with a focus on a measurement assembly accommodation portion 1106 which accommodates the measurement assembly 1200.

The measurement container 1100 of the turbidity monitoring apparatus 20 according to the second embodiment of the disclosure may be surrounded by the housing 1103 and have formed therein a fluid accommodation portion 1104 which accommodates a fluid to be measured is formed. The measurement container 1100 may include an inlet pipe 1101 which supplies fluid to the fluid accommodation portion 1104 and an outlet pipe 1102 which discharges fluid to the outside.

From another perspective, the housing 1103 may have formed therein the fluid accommodation portion 1104 which accommodates the fluid to be measured.

In addition, the fluid accommodation portion 1104 may include a space which has a certain volume and accommodates and allows fluid to flow from the inlet pipe 1101.

In this case, the housing 1103, the inlet pipe 1101, and the outlet pipe 1102 may be integrally formed as a single body.

From another perspective, an inner surface of the inlet pipe 1101 and an inner surface of the fluid accommodation portion 1104 may be connected to each other, and the inner surface of the fluid accommodation portion 1104 and an inner surface of the outlet pipe 1102 may also be connected to each other. Specifically, the housing 1103, the inlet pipe 1101, and the outlet pipe 1102 may be formed as a single conduit.

From another perspective, a central axis of the inlet pipe 1101 and the outlet pipe 1102 may coincide with a central axis of the housing 1103.

From another perspective, in the measurement container 1100 according to the second embodiment may have a separate inlet pipe 1101 and outlet pipe 1102 omitted.

Unlike the measurement container 1100 according to the first embodiment described above, in the second embodiment, the housing 1103 may perform the roles of the inlet pipe 1101 and the outlet pipe 1102. For example, the housing 1103 may have an inlet portion provided at an end thereof and an outlet portion provided at another end thereof.

In other words, the fluid accommodation portion 1104 may be formed between the inlet pipe 1101 and the outlet pipe 1102, not as a space having a diameter larger than that of the inlet pipe 1101 and the outlet pipe 1102, but as a space having a same diameter as the inlet pipe 1101 and the outlet pipe 1102.

Therefore, a fluid to be measured supplied from an external fluid supply pipe (for example, a water pipe) may directly flow into the housing 1103 and pass through the fluid accommodation portion 1104.

For example, the inlet pipe 1101 may be connected to the water pipe, and the outlet pipe 1102 may be connected to a water meter. In other words, a side of the housing 1103, which functions as an inlet pipe, may be connected to the water pipe, and another side of the housing 1103, which functions as an outlet pipe, may be connected to the water meter.

As another example, a water meter connector 1107 may be coupled to the outlet pipe 1102. For example, the housing 1103 and the water meter may be connected via the water meter connector 1107.

Meanwhile, the wave source 1210 and the detector 1220 may be positioned together on a side of the measurement container 1100. Specifically, the wave source 1210 and the detector 1220 may be positioned together on a side of the housing 1103 where the fluid accommodation portion 1104 is formed.

The measurement container 1100 may include the measurement assembly accommodation portion 1106 which accommodates the measurement assembly 1200. In this case, the measurement assembly accommodation portion 1106 may be positioned on a side surface of the housing 1103.

For example, the wave source 1210 and the detector 1220 described above may be accommodated in the measurement assembly accommodation portion 1106. The measurement assembly 1200 will be described in detail below.

The measurement assembly accommodation portion 1106 may be integrally formed as a single body with the housing 1103. The spirit of the disclosure is not limited thereto, and the measurement assembly accommodation portion 1106 may be formed as a separate member from the housing 1103 and coupled to the housing 1103.

The measurement assembly accommodation portion 1106 may be formed as a hollow box from which a side surface (upper surface) is removed, and the measurement assembly 1200 may be accommodated therein for assembly.

Referring further to FIGS. 15 and 16, the measurement container 1100 may include an opening 1105 penetrating both the housing 1103 and the measurement assembly accommodation portion 1106.

For example, the opening 1105 may be formed at a portion in which the housing 1103 is in contact with the measurement assembly accommodation portion 1106. In this case, the opening 1105 may be a portion in which a plate 1231 of the measurement assembly 1200, which will be described below, is positioned. In other words, the opening 1105 may be a portion in which the wave source 1210 and the detector 1220 positioned on the plate 1231 are positioned.

From another perspective, the measurement assembly accommodation portion 1106 may include a bottom portion 1106a, and the bottom portion 1106a may be formed with the opening 1105 in communication with the housing 1103. In addition, the plate 1231 may be positioned on the bottom portion 1106a to cover the opening 1105.

The opening 1105 may be formed to have a certain length and width. In this case, a longitudinal direction of the opening 1105 may be identical to a longitudinal direction of the housing 1103, and a width direction of the opening 1105 may be perpendicular to the longitudinal direction of the housing 1103.

In addition, a width W3 of the opening 1105 may be smaller than an inner diameter ID of the housing 1103, and a length L4 of the opening 1105 may be greater than the width W3 of the opening 1105. However, the spirit of the disclosure is not limited thereto, and the length of the opening 1105 may be smaller than the width thereof, and a size of the opening 1105 may vary in various manners.

In addition, the opening 1105 may form a first space S1 having a certain depth corresponding to a distance from an inner surface of the housing 1103 to a bottom surface of the measurement assembly accommodation portion 1106. For example, the housing 1103 may have a certain depth from the inner surface forming the fluid accommodation portion 1104 to a bottom portion of the measurement assembly accommodation portion 1106.

Referring again to FIGS. 14 and 16, the depth of the first space S1 may vary depending on the width direction of the opening 1105. Specifically, a shortest distance from the inner surface of the housing 1103 to the plate 1231 may be denoted as D1, and D1 may correspond to a thickness of the housing 1103 at a portion in which the measurement assembly accommodation portion 1106 is positioned.

Furthermore, the opening 1105 may include a side wall corresponding to the longitudinal direction of the housing 1103, and a depth corresponding to the side wall may be denoted as D3. Also, a distance of a deepest area formed by the opening 1105 may be denoted as D3.

For example, the first space S1 formed by the opening 1105 may exhibit a difference in distance of D2 between the deepest area and a shallower area.

From another perspective, the fluid accommodation portion 1104 according to an embodiment of the disclosure may be in a shape in which the first space S1 formed by the opening 1105 is further expanded on a second space S2 of a tubular form.

Also, the fluid to be measured flowing inside the housing 1103 may flow not only through the second space S2, but also into the first space S1 formed by the opening 1105.

In other embodiments, the measurement assembly 1200 may include a case 1230 which accommodates the wave source 1210 and the detector 1220. Although the case 1230 shown in the drawings is in a shape corresponding to the measurement assembly accommodation portion 1106, the spirit of the disclosure is not limited thereto, and the case 1230 may be formed in various shapes which accommodate the wave source 1210 and the detector 1220.

In addition, the case 1230 may include the plate 1231 positioned adjacent to the fluid accommodation portion 1104. Specifically, the plate 1231 may include a bottom surface of the case 1230. For example, the plate 1231 may be integrally formed as a single body with the case 1230, and a bottom portion of the case 1230 may be referred to as the plate 1231. In other embodiments, the plate 1231 may be formed as a member separate from the case 1230 and may be coupled to the case 1230.

In this case, the plate 1231 may include a same material as the case 1230, or may include a different material from the case 1230.

Specifically, the plate 1231 may include a light-transmitting area. In an embodiment, the plate 1231 may have an overall light-transmitting area. In other words, the plate 1231 may include a transparent material.

The plate 1231 including the light-transmitting area may be positioned under the wave source 1210 and the detector 1220 such that a wave irradiated from the wave source 1210 may reach the fluid accommodation portion 1104, and the detector 1220 may detect a speckle pattern formed in the fluid accommodation portion 1104.

In case that a surface of the plate 1231 opposite the measurement assembly accommodation portion 1106 is referred to as an outer surface of the plate 1231, a sealing member 1232 may be coupled to the outer surface of the plate 1231.

From another perspective, the sealing member 1232 may be positioned between the bottom portion 1106a of the measurement assembly accommodation portion 1106 and the plate 1231. Specifically, a groove 1106b into which the sealing member 1232 may be inserted may be formed in the bottom portion 1106a of the measurement assembly accommodation portion 1106.

The groove 1106b may be formed along a periphery of the opening 1105. Also, the sealing member 1232 may be formed in a shape corresponding to the groove 1106b.

As described above, by positioning the sealing member 1232 under the plate 1231, in case that the measurement assembly 1200 is accommodated in the measurement assembly accommodation portion 1106, the sealing member 1232 may be fitted into the groove 1106b, thereby sealing a space between the plate 1231 and the bottom portion 1106a.

Accordingly, leakage of the fluid to be measured from the fluid accommodation portion 1104 through the opening 1105 may be restricted.

In other embodiments, the wave source 1210 and the detector 1220 may be accommodated in the case 1230 in a state in which the wave source 1210 and the detector 1220 are coupled to a control circuit 1240. In this case, the control circuit 1240 may include a controller (not shown) as described above.

As described above, the measurement assembly 1200 may be treated as a unit part in which the wave source 1210 and the detector 1220 are coupled with the case 1230. Accordingly, the turbidity monitoring apparatus 20 may be manufactured by assembling the measurement assembly 1200 into the measurement assembly accommodation portion 1106 of the housing 1103.

Hereinafter, a turbidity monitoring apparatus 30 according to a third embodiment of the disclosure will be described. In this case, the turbidity monitoring apparatus according to the third embodiment of the disclosure differs from the turbidity monitoring apparatus 10 according to the first embodiment of the disclosure described above with respect to the configuration of a measurement container.

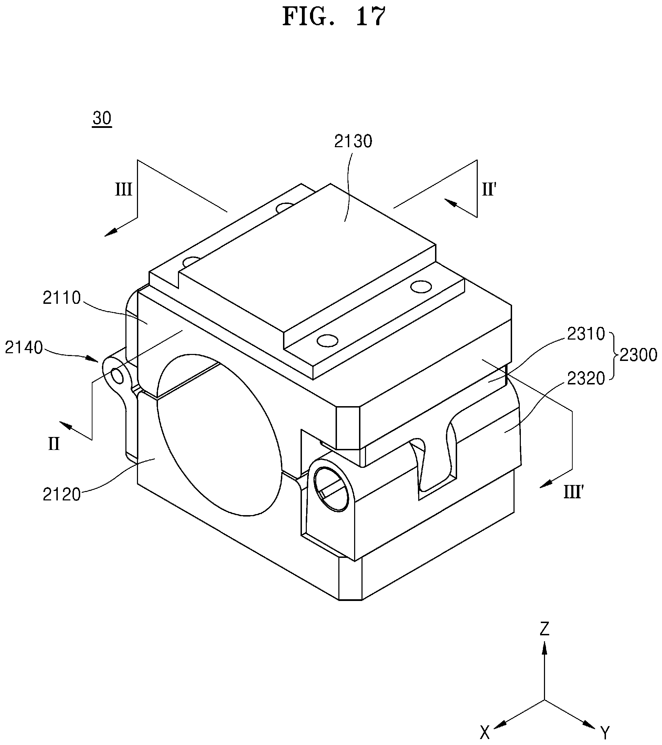

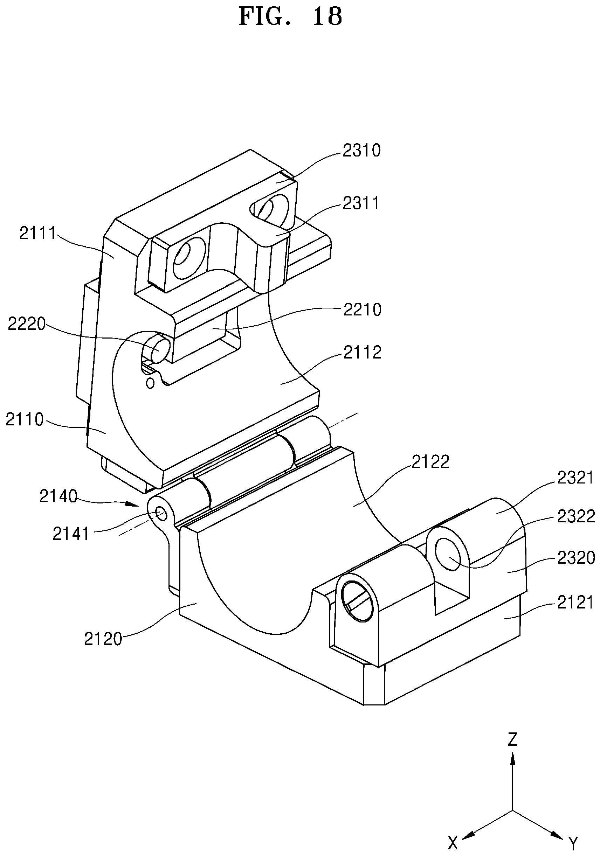

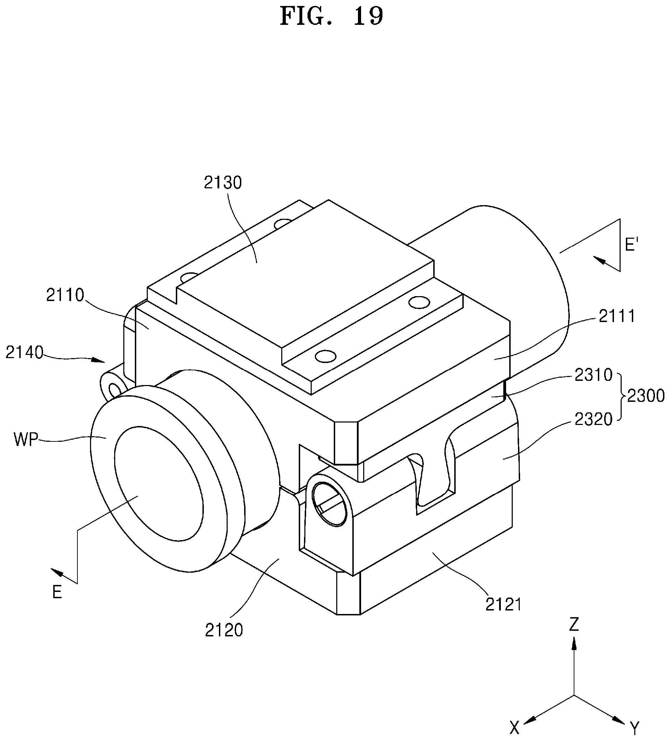

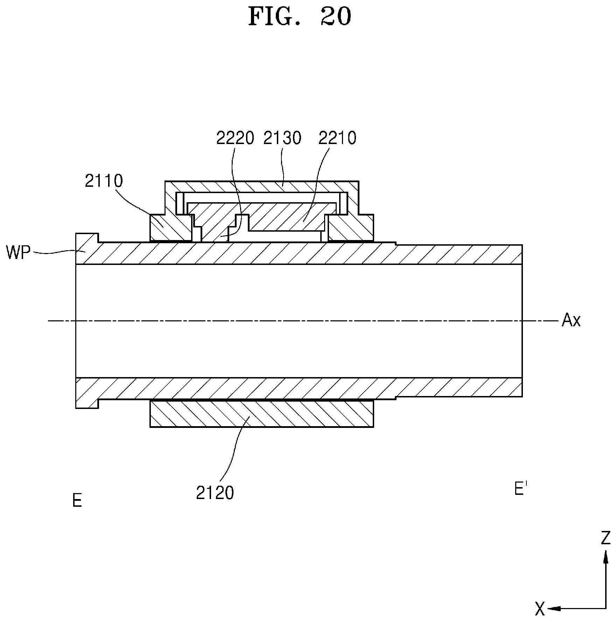

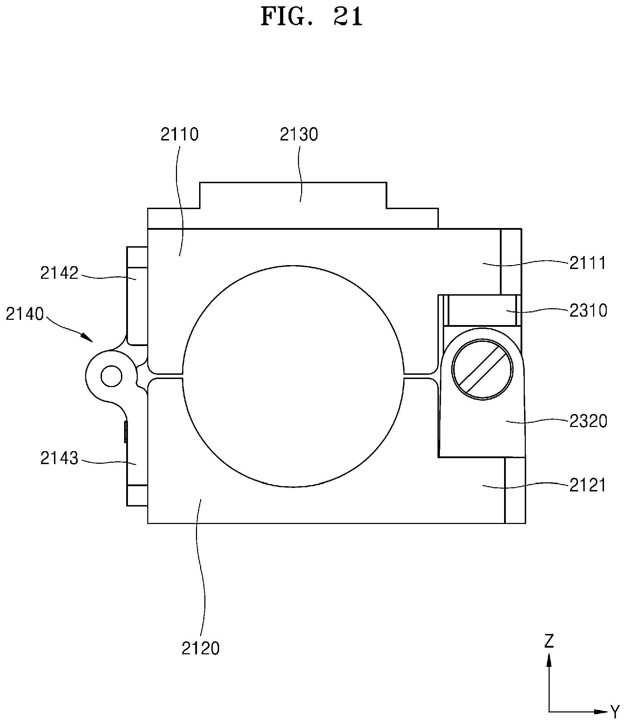

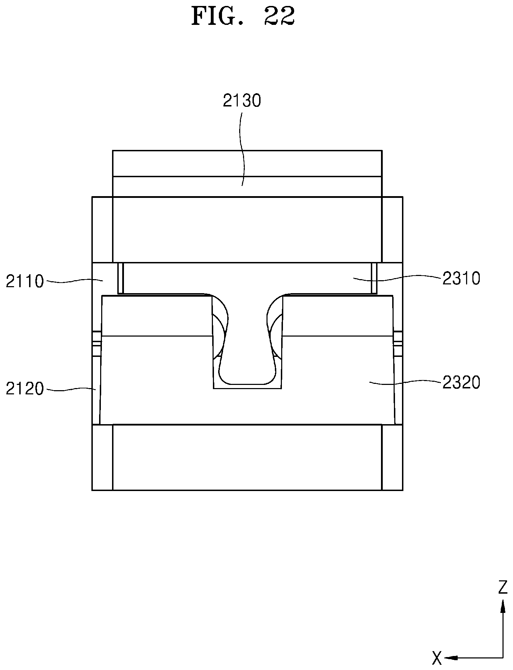

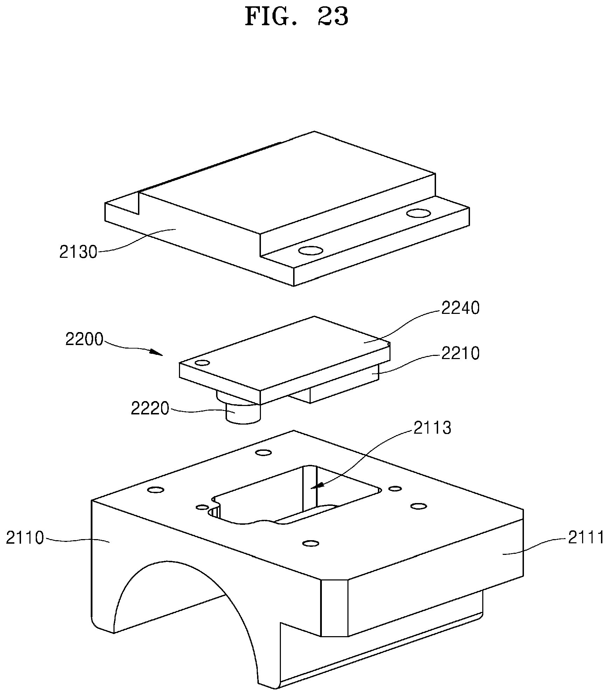

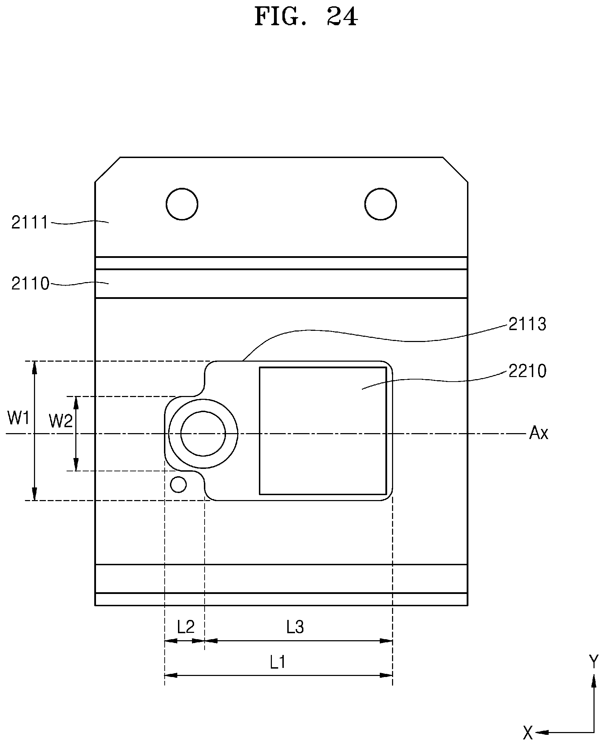

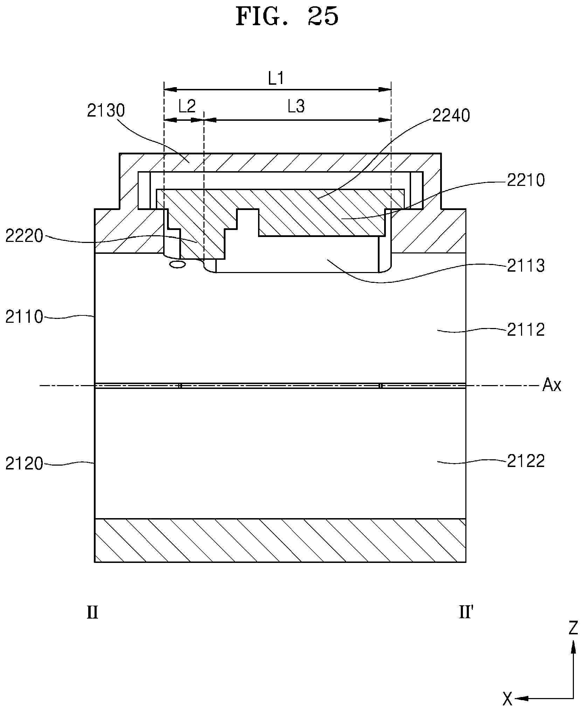

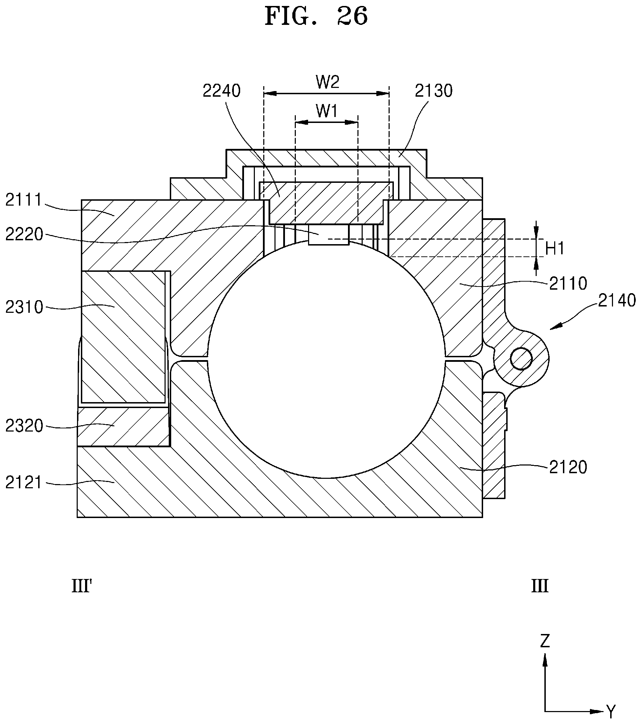

FIG. 17 is a perspective view of the turbidity monitoring apparatus 30 according to the third embodiment of the disclosure, and FIG. 18 is a perspective view of the turbidity monitoring apparatus 30 of FIG. 17 in an opened state. FIG. 19 is a perspective view illustrating a state in which the turbidity monitoring apparatus 30 of FIG. 17 is coupled to a conduit, and FIG. 20 is a cross-sectional view of the conduit taken along a line E-E' of FIG. 19. FIG. 21 is a front view of the turbidity monitoring apparatus 30 of FIG. 17 from a different angle, and FIG. 22 is a side view of the turbidity monitoring apparatus 30 of FIG. 17 from a different angle. FIG. 23 is an exploded perspective view of a first body portion 2110 and some components of the turbidity monitoring apparatus 30 of FIG. 17 in an exploded state, and FIG. 24 is a plan view illustrating a state in which the first body portion 2110 and a measurement assembly 2200 of the turbidity monitoring apparatus 30 of FIG. 17 are coupled to each other. FIG. 25 is a cross-sectional view of the turbidity monitoring apparatus 30 taken along a line II-II' of FIG. 17, and FIG. 26 is a cross-sectional view of the turbidity monitoring apparatus 30 taken along a line III-III' of FIG. 17.

Referring to FIGS. 17 to 22, the turbidity monitoring apparatus 30 according to the third embodiment of the disclosure may include the first body portion 2110, a second body portion 2120, and the measurement assembly 2200. In this case, the measurement assembly 2200 may include a wave source 2210 and a detector 2220. In addition, although not shown in the drawings, the turbidity monitoring apparatus 30 may further include a controller (not shown).

Because the wave source 2210 and the detector 2220 according to the third embodiment of the disclosure are substantially identical to the wave source 210 and the detector 220 described in the first embodiment, a detailed description thereof will be omitted herein.

Hereinafter, the first body portion 2110 and the second body portion 2120 of the third embodiment of the disclosure will be described in more detail with a focus on a coupling structure of the measurement assembly 2200.

The turbidity monitoring apparatus 30 according to the third embodiment of the disclosure may be an apparatus provided separately from a conduit WP through which a fluid to be measured flows, and may be mounted on the conduit WP to measure the turbidity of the fluid flowing through the conduit WP.

In this case, the conduit WP may be a transparent conduit and may include a material through which waves irradiated from the wave source 2210 may pass.

The turbidity monitoring apparatus 30 according to the third embodiment of the disclosure may include the first body portion 2110, the second body portion 2120, and the measurement assembly 2200.

The first body portion 2110 may be positioned to surround at least a portion of the conduit WP through which the fluid to be measured flows.

Specifically, the first body portion 2110 may include a first surface 2112 corresponding to an outer circumferential surface of the conduit WP and may be positioned in close contact with the outer circumferential surface of the conduit WP.

The second body portion 2120 may be positioned to surround at least a portion of the conduit WP and may be separated from or coupled to the first body portion 2110.

In case that the turbidity monitoring apparatus 30 according to an embodiment is mounted on the conduit WP, the first body portion 2110 and the second body portion 2120 may be positioned to face each other with respect to the conduit WP.

Specifically, the first body portion 2110 and the second body portion 2120 may be formed symmetrically. For example, the first body portion 2110 and the second body portion 2120 may each be formed to surround a respective half of the outer circumferential surface of the conduit WP.

The turbidity monitoring apparatus 30 according to the third embodiment may further include a hinge portion 2140 and a fastening portion 2300.

In this case, the hinge portion 2140 may include a first fixing portion 2142 and a second fixing portion 2143, which are shaft-coupled to be relatively rotatable about a single axis. For example, the hinge portion 2140 may include a hinge shaft 2141.

In addition, the first body portion 2110 and the second body portion 2120 may be connected to each other via the hinge portion 2140 including the hinge shaft 2141 described above.

Specifically, the first fixing portion 2142 of the hinge portion 2140 may be coupled to the first body portion 2110, and the second fixing portion 2143 may be coupled to the second body portion 2120.

Accordingly, the first body portion 2110 and the second body portion 2120 may be connected to be relatively rotatable about a single axis.

However, the hinge portion 2140 shown in the drawings is merely an example, and the scope of the disclosure is not limited thereto. Various structures which allow the first body portion 2110 and the second body portion 2120 to be rotatably connected to each other are also possible.

In other embodiments, the fastening portion 2300 may be positioned on a side opposite the hinge portion 2140. The first body portion 2110 and the second body portion 2120 may be detachably coupled to each other through the fastening portion 2300.

Specifically, the fastening portion 2300 may include a first fastening member 2310 coupled to the first body portion 2110 and a second fastening member 2320 coupled to the second body portion 2120.

For example, the fastening portion 2300 may have a ball catch structure. Specifically, the first fastening member 2310 may be a catch plate including a protrusion, and the second fastening member 2320 may be a ball housing including balls.

In this case, a protrusion 2311 of the catch plate may be positioned between two balls 2321 provided in a ball housing 2322 such that the balls 2321 are fitted into a groove of the protrusion 2311, and accordingly, the two fastening portions (first and second fastening members 2310 and 2320) may be fixed to each other.

In other embodiments, the first body portion 2110 may be a portion to which the first fastening member 2310 is coupled, and may include a first extension portion 2111. Also, the second body portion 2120 may be a portion to which the second fastening member 2320 is coupled, and may include a second extension portion 2121. In this case, the first extension portion 2111 and the second extension portion 2121 may be formed to extend respectively from the first body portion 2110 and the second body portion 2120, such that the first extension portion 2111 and the second extension portion 2121 are parallel to each other.

As described above, in the turbidity monitoring apparatus 30 according to the third embodiment of the disclosure, a side of the first body portion 2110 and a side of the second body portion 2120 are connected to each other via the hinge portion 2140, and the first body portion 2110 and the second body portion 2120 may rotate to be closer to or move away from each other.

In addition, the fastening portion 2300 may be coupled to another side of the first body portion 2110 and another side of the second body portion 2120 such that the first fastening member 2310 and the second fastening member 2320 may be coupled to each other in a close state in which the first body portion 2110 and the second body portion 2120 are in closest proximity. For example, in a state in which the first body portion 2110 and the second body portion 2120 surround the conduit WP and are in a closed state, the first fastening member 2310 and the second fastening member 2320 may be coupled to each other such that the first body portion 2110 and the second body portion 2120 may be stably fixed to the conduit WP.

Referring also to FIGS. 23 to 26, the first body portion 2110 or the second body portion 2120 may form an opening 2113 corresponding to a portion in which the measurement assembly 2200 is positioned.

In the third embodiment, a case where the opening 2113 is formed in the first body portion 2110 will be described.

The opening 2113 may be a portion penetrating in an outward direction away from the conduit WP from the first surface 2112 of the first body portion 2110.

From another perspective, the opening 2113 may be a portion penetrating from an outermost portion of the first body portion 2110 to the first surface 2112 which is in contact with the outer circumferential surface of the conduit WP.

The opening 2113 may be formed to correspond to a size and structure of the measurement assembly 2200. Specifically, the measurement assembly 2200 may include the wave source 2210 and the detector 2220 as described above. In addition, the wave source 2210 and the detector 2220 may be assembled while being positioned in a control circuit.

In this case, the wave source 2210 and the detector 2220 may be sequentially positioned in a direction parallel to a longitudinal direction of the conduit WP. Specifically, the wave source 2210 and the detector 2220 may be sequentially positioned in a flow direction of the fluid to be measured in the conduit WP.

In this case, the measurement assembly 2200 may be positioned overall to be parallel to the longitudinal direction of the conduit WP.

Accordingly, the opening 2113 may be formed in an elongated shape in the longitudinal direction of the conduit WP to correspond to a direction in which the measurement assembly 2200 is positioned. For example, a longitudinal direction of the opening 2113 may be parallel to the longitudinal direction of the conduit WP.

In other words, the opening 2113 may have a distance in a longitudinal direction greater than a distance in a width direction. For example, a length L1 of the opening 2113 may be greater than a width W1 of the opening 2113.

In addition, the opening 2113 may be formed such that a shape of a portion in which the wave source 2210 is positioned differs from a shape of a portion in which the detector 2220 is positioned.

For example, a width W1 of the portion in which the wave source 2210 is positioned may be greater than a width W2 of the portion in which the detector 2220 is positioned.

Specifically, the opening 2113 may have an overall wide area but may include a portion in which the width is narrower. In addition, a length L2 of the portion in which the width is narrower around the detector 2220 may be shorter than a length L3 of a remaining area of the opening 2113.

As described above, the width of the opening 2113 may be formed to correspond to a size of the detector 2220, thereby reducing an empty space around the detector 2220. Accordingly, the detector 2220 may be protected from external impact in a state in which the measurement assembly 2200 is mounted to the first body portion 2110.

The measurement assembly 2200 may be positioned in the first body portion 2110 or the second body portion 2120. For example, the wave source 2210 and the detector 2220 may be positioned together on one of the first body portion 2110 or the second body portion 2120. In addition, the measurement assembly 2200 may be covered with a cover 2130.

In the third embodiment, a case where the wave source 2210 and the detector 2220 are positioned together on the first body portion 2110 will be described.

As described above, the opening 2113 may be formed in the first body portion 2110, and the measurement assembly 2200 may be positioned to correspond to the opening 2113. In this case, a portion of the wave source 2210 may be positioned to protrude into the opening 2113. Similarly, a portion of the detector 2220 may be positioned to protrude into the opening 2113.

For example, the opening 2113 may be a portion which accommodates components of the measurement assembly 2200, such as the wave source 2210 and the detector 2220.

The measurement assembly 2200 may measure the turbidity of fluid flowing through the transparent conduit in a state in which the first body portion 2110 is fixed to the transparent conduit.

Specifically, because the wave source 2210 and the detector 2220 are positioned to face the transparent conduit, as described in the first embodiment, the wave source 2210 may irradiate a wave toward the fluid flowing through the transparent conduit, and a speckle pattern may be detected by the detector 2220 to measure the turbidity of the fluid to be measured.

As described above, according to the third embodiment of the disclosure, the turbidity of the fluid to be measured may be measured by installing the turbidity monitoring apparatus 30 on a transparent conduit provided in the related art.

The turbidity monitoring apparatus 30 including the first body portion 2110 and the second body portion 2120 may be easily attached to and detached from the transparent conduit, thereby improving portability and usability.

Hereinafter, a turbidity monitoring apparatus 40 according to a fourth embodiment of the disclosure will be described. In this case, the turbidity monitoring apparatus 40 according to the fourth embodiment of the disclosure differs from the turbidity monitoring apparatus 30 according to the third embodiment of the disclosure described above with respect to the configuration of a second body portion 3120.

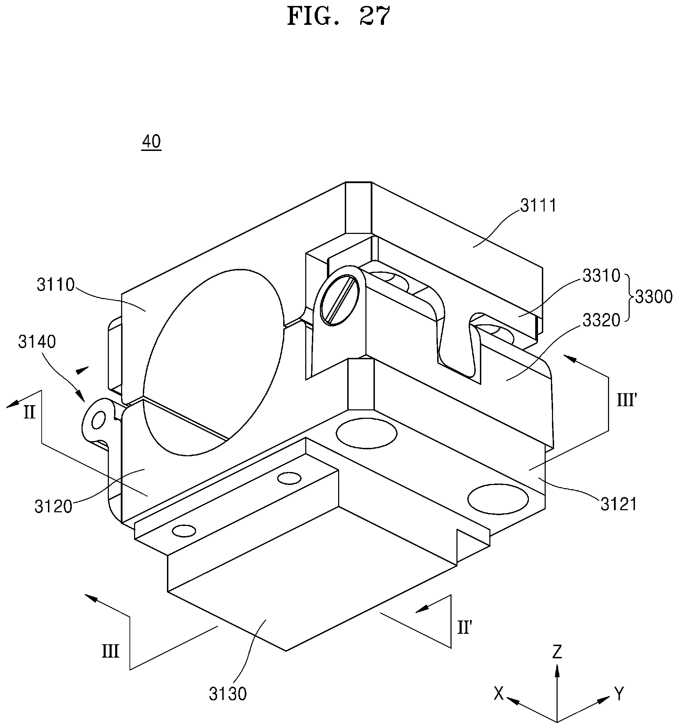

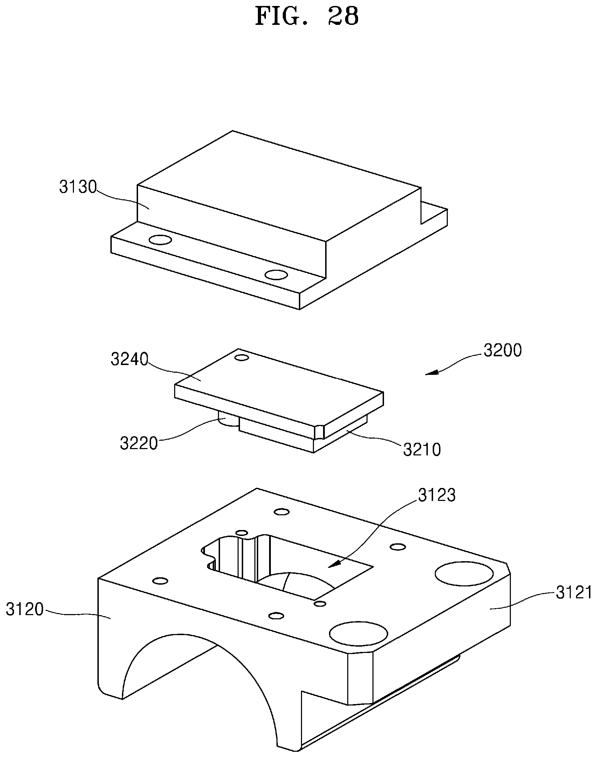

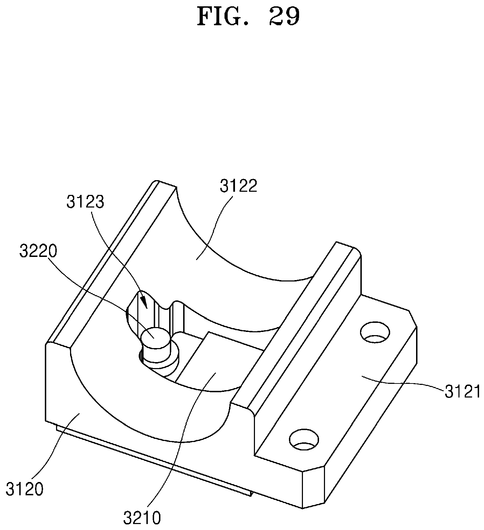

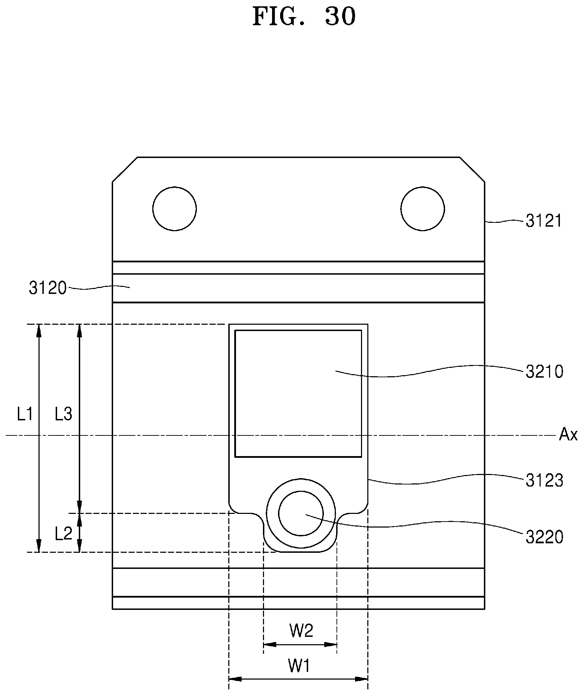

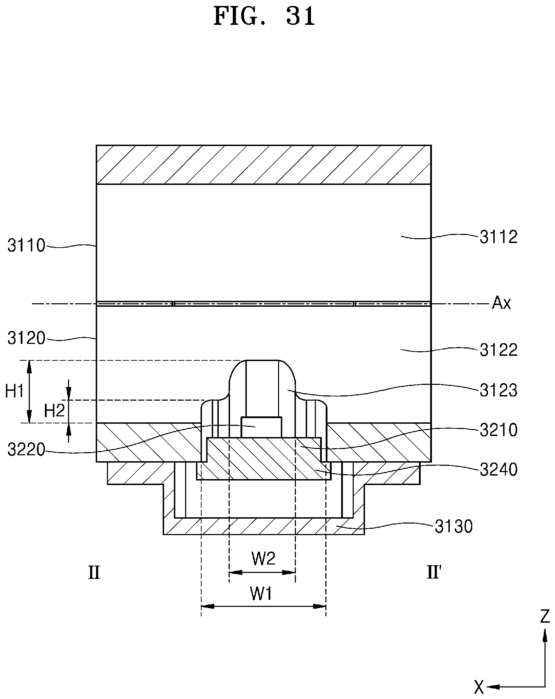

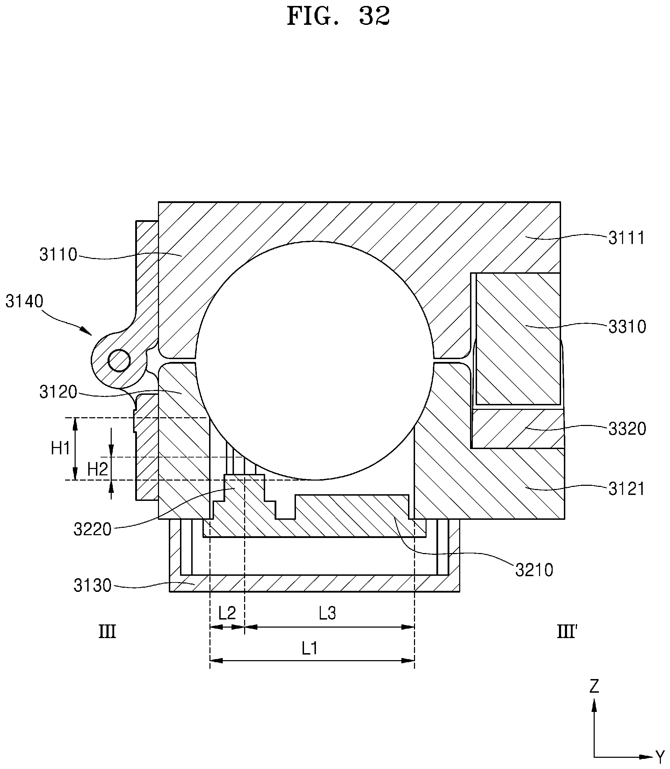

FIG. 27 is a perspective view of the turbidity monitoring apparatus 40 according to the fourth embodiment of the disclosure, and FIG. 28 is an exploded perspective view of the second body portion 3120 and some components of the turbidity monitoring apparatus 40 of FIG. 27 in an exploded state. FIG. 29 is a perspective view illustrating a state in which the second body portion 3120 and a measurement assembly 3200 of the turbidity monitoring apparatus 40 of FIG. 27 are coupled to each other, and FIG. 30 is a plan view illustrating a state in which the second body portion 3120 and the measurement assembly 3200 of the turbidity monitoring apparatus 40 of FIG. 27 are coupled to each other. FIG. 31 is a cross-sectional view of the turbidity monitoring apparatus 40 taken along a line II-II' of FIG. 27, and FIG. 32 is a cross-sectional view of the turbidity monitoring apparatus 40 taken along a line III-III' of FIG. 27

Referring to FIGS. 27 to 32, the turbidity monitoring apparatus 40 according to the fourth embodiment of the disclosure may include a first body portion 3110, the second body portion 3120, and the measurement assembly 3200.

Because a wave source 3210 and a detector 3220 according to the fourth embodiment of the disclosure are substantially identical to the wave source 210 and the detector 220 described in the first embodiment, a detailed description thereof will be omitted herein.

Also, the first body portion 3110, the second body portion 3120, the measurement assembly 3200, a hinge portion 3140, and a fastening portion 3300 are substantially identical to those described in the third embodiment to the corresponding extent, and thus, detailed descriptions thereof will also be omitted herein.

Hereinafter, the second body portion 3120 of the fourth embodiment of the disclosure will be described in more detail with a focus on a coupling structure of an opening 3123 and the measurement assembly 3200.

The measurement assembly 3200 may be positioned in the first body portion 3110 or the second body portion 3120. The wave source 3210 and the detector 3220 may also be positioned together on one of the first body portion 3110 or the second body portion 3120.

The first body portion 3110 or the second body portion 3120 may form the opening 3123 to correspond to a portion in which the measurement assembly 3200 is positioned.

In the fourth embodiment, a case where the wave source 3210 and the detector 3220 are positioned together on the second body portion 3120 will be described. Specifically, in the fourth embodiment, a case where the opening 3123 is formed in the second body portion 3120 will be described.

The opening 3123 may be a portion penetrating in an outward direction away from a conduit from a second surface 3122 of the second body portion 3120.

From another perspective, the opening 3123 may be a portion penetrating from an outermost portion of the second body portion 3120 to the second surface 3122 which is in contact with an outer circumferential surface of the conduit.

The opening 3123 may be formed to correspond to a size and structure of the measurement assembly 3200. Specifically, the measurement assembly 3200 may include the wave source 3210 and the detector 3220 as described above. In addition, the wave source 3210 and the detector 3220 may be assembled while being positioned in a control circuit 3240.

In this case, the wave source 3210 and the detector 3220 may be sequentially positioned in a direction that is not parallel to a longitudinal direction of the conduit.

In an embodiment, the wave source 3210 and the detector 3220 may be sequentially positioned in a direction perpendicular to the longitudinal direction of the conduit. Specifically, the wave source 3210 and the detector 3220 may be positioned in a direction perpendicular to a direction in which a fluid to be measured flows in the conduit.

In this case, the measurement assembly 3200 may be positioned overall to be perpendicular to the longitudinal direction of the conduit.

Accordingly, the opening 3123 may be formed in an elongated shape in a width direction of the conduit to correspond to a direction in which the measurement assembly 3200 is positioned. For example, a longitudinal direction of the opening 3123 may be parallel to the width direction of the conduit. Specifically, the longitudinal direction of the opening 3123 may cross the longitudinal direction of the conduit.

From another perspective, the width direction of the opening 3123 may correspond to the longitudinal direction of the conduit.

The opening 3123 may have a length L1 greater than a width W1 of the opening 3123.

In addition, the opening 3123 may be formed such that a shape of a portion in which the wave source 3210 is positioned differs from a shape of a portion in which the detector 3220 is positioned.

For example, the width W1 of the portion in which the wave source 3210 is positioned may be greater than a width W2 of the portion in which the detector 3220 is positioned.

Specifically, the opening 3123 may have an overall wide area but may include a portion in which the width is narrower. In addition, a length L2 of the portion in which the width is narrower around the detector 3220 may be shorter than a length L3 of a remaining area of the opening 3123.

Furthermore, a depth of the opening 3123 in the portion in which the detector 3220 is positioned may vary depending on the position. For example, in case that a diameter direction of the conduit, which is parallel to the first body portion 3110 and the second body portion 3120 is assumed to be in the longitudinal direction of the opening 3123, the depth of the opening 3123 may vary depending on the longitudinal direction of the opening 3123. For example, referring to the drawing shown in FIG. 32, in case that a Z-axis distance is measured from a lowest point on a second surface among various points, a height of a narrowed portion of the opening 3123 may vary from H2 to H1.

As described above, the width and depth of the opening 3123 may be formed to correspond to a size of the detector 3220, thereby reducing an empty space around the detector 3220. Accordingly, the detector 3220 may be protected from external impact in a state in which the measurement assembly 3200 is mounted in the second body portion 3120.

As described above, the opening 3123 may be formed in the second body portion 3120, and the measurement assembly 3200 may be positioned to correspond to the opening 3123. In this case, a portion of the wave source 3210 may be positioned to protrude into the opening 3123. Similarly, a portion of the detector 3220 may be positioned to protrude into the opening 3123.

For example, the opening 3123 may be a portion which accommodates components of the measurement assembly 3200, such as the wave source 3210 and the detector 3220.

The measurement assembly 3200 may measure the turbidity of fluid flowing through the transparent conduit in a state in which the second body portion 3120 is fixed to the transparent conduit.

In other embodiments, although a case where the measurement assembly 3200 is coupled to the first body portion 3110 or the second body portion 3120 has been described as an example in the description of the disclosure, the spirit of the disclosure is not limited thereto, and the measurement assembly 3200 may be positioned in each of the first body portion 3110 and the second body portion 3120, and the wave source 3210 may be positioned in the first body portion 3110 while the detector 3220 may be positioned in the second body portion 3120.

In addition, because the wave source 3210 and the detector 3220 are positioned to face the transparent conduit, as described in the first embodiment, the wave source 3210 may irradiate a wave toward the fluid flowing through the transparent conduit, and a speckle pattern may be detected by the detector 3220 to measure the turbidity of the fluid to be measured.

As described above, according to the fourth embodiment of the disclosure, the turbidity of the fluid to be measured may be measured by installing the turbidity monitoring apparatus 40 on a transparent conduit provided in the related art.

The turbidity monitoring apparatus 40 including the first body portion 3110 and the second body portion 3120 may be easily attached to and detached from the transparent conduit, thereby improving portability and usability.

As described above, the disclosure has been described with reference to the embodiments shown in the accompanying drawings, but should be considered in a descriptive sense only. Those of ordinary skill in the art will understand that various modifications and changes to the embodiments may be made therefrom. Therefore, the true technical scope of protection of the disclosure should be defined by the technical spirit of the appended claims.

### Industrial Applicability

Provided is a turbidity monitoring apparatus having a structure which facilitates removal of bubbles generated in a measurement space of the turbidity monitoring apparatus

## Claims

1. A turbidity monitoring apparatus comprising:
a measurement container in which a fluid accommodation portion configured to accommodate a fluid to be measured is formed, the measurement container including an inlet pipe configured to supply the fluid to be measured to the fluid accommodation portion, and an outlet pipe configured to discharge the fluid to be measured to the outside;
a wave source configured to irradiate a wave toward the fluid accommodation portion; and
a detector configured to detect a laser speckle generated by multiple scattering of the irradiated wave in the fluid to be measured,
wherein the measurement container includes
a flow path formation portion formed on a side wall surface of the fluid accommodation portion, and configured to guide a flow of the fluid to be measured introduced through the inlet pipe, and
the wave source and the detector are positioned adjacent to a surface of the measurement container on which the flow path formation portion is formed.

2. The turbidity monitoring apparatus of claim 1, wherein
a central axis of the inlet pipe and a central axis of the outlet pipe are parallel to each other.

3. The turbidity monitoring apparatus of claim 2, wherein
the flow path formation portion is positioned in parallel with the inlet pipe and the outlet pipe.

4. The turbidity monitoring apparatus of claim 1, wherein
a central axis of the inlet pipe is closer to the flow path formation portion than a central axis of the outlet pipe.

5. The turbidity monitoring apparatus of claim 1, wherein
the wave source is positioned closer to an inlet portion of the inlet pipe than the detector.

6. The turbidity monitoring apparatus of claim 1, wherein
the flow path formation portion includes:
a planar portion;
a first curved portion extending from the planar portion toward the outlet pipe to form a curved surface; and
a second curved portion extending from the planar portion toward the inlet pipe to form a curved surface.

7. The turbidity monitoring apparatus of claim 6, wherein
the detector and the wave source are positioned on a side of the planar portion.

8. The turbidity monitoring apparatus of claim 6, wherein
the first curved portion is formed to have a longer curved surface length than the second curved portion.

9. The turbidity monitoring apparatus of claim 1, wherein
the flow path formation portion is configured to form a flow of the fluid to be measured in the fluid accommodation portion to reduce bubble generation in the fluid accommodation portion and to reduce bubbles remaining in the fluid accommodation portion.

10. A turbidity monitoring apparatus comprising:
a measurement container in which a housing having a fluid accommodation portion formed therein to accommodate a fluid to be measured, an inlet pipe configured to supply the fluid to be measured, and an outlet pipe configured to discharge the fluid to be measured to the outside are integrally formed as a single body; and
a measurement assembly including a wave source configured to irradiate a wave toward the fluid accommodation portion, and a detector configured to detect a laser speckle generated by multiple scattering of the irradiated wave in the fluid to be measured,
wherein central axes of the inlet pipe and the outlet pipe coincide with a central axis of the housing, and
the wave source and the detector are positioned together on a side of the measurement container.

11. The turbidity monitoring apparatus of claim 10, wherein
the housing, the inlet pipe, and the outlet pipe are formed as a single conduit.

12. The turbidity monitoring apparatus of claim 10, wherein
the measurement container includes
a measurement assembly accommodation portion positioned on a side surface of the housing to accommodate the measurement assembly.

13. The turbidity monitoring apparatus of claim 12, wherein
the measurement container includes an opening penetrating the housing and the measurement assembly accommodation portion.

14. The turbidity monitoring apparatus of claim 13, wherein
the opening is configured to form a first space having a certain depth corresponding to a distance from an inner surface of the housing to a bottom surface of the measurement assembly.

15. The turbidity monitoring apparatus of claim 13, wherein
a longitudinal direction of the opening is identical to a longitudinal direction of the housing, and
a width of the opening is smaller than an inner diameter of the housing, and a length of the opening is greater than the width of the opening.

16. The turbidity monitoring apparatus of claim 10, wherein
the measurement assembly includes a case configured to accommodate the wave source and the detector, and
the case includes a plate positioned adjacent to the fluid accommodation portion.

17. The turbidity monitoring apparatus of claim 16, wherein
the plate includes a light-transmitting area.
